# EUROPEAN PATENT APPLICATION

(11) **EP 1 600 454 A1**
(43) Date of publication of application: **30.11.2005**
(21) Application number: 03703370.1
(22) Date of filing: 25.02.2003
(51) Int. Cl.: C07H 17/07, A61K 31/7048

(54) **ANTI-INFLUENZA VIRUS COMPOUND COMPRISING BIFLAVONOID-SIALIC ACID GLYCOSIDE**

(71) Applicant: THE KITASATO INSTITUTE, Tokyo 108-8642 (JP)
(72) Inventor: YAMADA, Haruki, Nerima-ku, Tokyo 177-0043 (JP); NAGAI, Takayuki, Setagaya-ku, Tokyo 156-0055 (JP); TAKAHASHI, Kunio, Kiyose-shi, Tokyo 204-8588 (JP)
(74) Representative: Grünecker, Kinkeldey, Stockmair & Schwanhäusser Anwaltssozietät
(86) International application number: PCT/JP2003/002046
(87) International publication number: WO 2004/076471

(57) **Abstract**

The compounds of the present invention comprise biflavonoid-sialic acid conjugates.

Such biflavonoid-sialic acid conjugates show anti-influenza virus activities, not only in *in vitro* systems using cultured cells, but also in *in vivo* systems using mice. Thus, they are useful as preventive or therapeutic agents for influenza, and moreover, are useful in food and drink products for preventing or treating influenza.

## Description

### Technical Field

This invention relates to antiviral compounds having sialidase inhibitory activities, which are useful for preventing or treating viral diseases including influenza.

### Background Art

Influenza is a serious life-threatening infectious disease for patients with underlying diseases or for elderly people. In fact, an increased excess rate of mortality is observed in years where there is an influenza epidemic. In addition, there are many reports of cases where concomitant pneumonia in elderly people and encephalitis in infants has led to death or serious conditions (Keizo Matsumoto, Nippon Rinsho 55 (10): 2536-2541 (1997)). Further, there has recently been concern about the emergence of a new subtype of influenza type A virus, and it is estimated that several tens of thousands to hundreds of thousands of people will die if it spreads in Japan. Thus, countermeasures against influenza are a socially important challenge. There is no doubt that preventive vaccinations are the most effective means for protection from influenza (Shin-ichi Tamura and Takeshi Kurata, BIO Clinica 11 (9): 665-669 (1996)). However, since vaccine production and supply in sufficient amounts requires a certain period of time, administration of anti-influenza agents is required to alleviate symptoms when a new viral epidemic is looming.

Presently, the anti-influenza virus drugs commercially available in Japan include amantadine, zanamivir, and oseltamivir. Health insurance covers amantadine for type A influenza virus infections, and zanamivir and oseltamivir for both type A and type B influenza virus infections. Amantadine acts on the influenza virus M2 protein. The M2 protein is an ion channel present on the membrane surface of type A influenza virus, and by acidifying the inside of the viral particle, plays a major role in releasing ribonucleoprotein complexes (RNP), which are RNA genes, nucleoproteins, and RNA polymerase complexes, into the cytoplasm. Amantadine inhibits the function of M2 protein, suppressing the release of RNPs, and preventing viral proliferation.

Zanamivir and oseltamivir are effective against both type A and B influenza viruses, and unlike amantadine, these agents exert their effect by inhibiting sialidase (also called neuraminidase). Sialidase is a glycoprotein present on the surface of influenza A and B viruses, and when proliferated viruses are released from host cells, it stimulates viral budding by cleaving hemagglutinin-receptor bonds (cleaving the sialic acid residues in the receptor) . Both zanamivir and oseltamivir inhibit sialidase activity by binding to its active site, thus preventing the release of viruses from host cells. Since the viruses trapped by the infected cells bind to other viruses, further spread of the infection is prevented, and the infection is eventually ended.

Zanamivir is also famous as a drug conceptually developed using computer chemistry. The drug emerged from progress in basic research that elucidated the structure and active site conformation of sialidase.

Oseltamivir also has inhibitory activity against sialidase function, strongly binding the active site of sialidase, which binds to sialic acid residues. Zanamivir must be inhaled as a powder using an inhaler or the like because of its low bioavailability when orally administered. On the other hand, oseltamivir is a prodrug that is converted to an active form after being absorbed by the body, and it can be administered orally.

Any of these anti-influenza drugs must be administered within 48 hours of disease onset. Administration more than 48 hours after onset has little impact, and does not change the natural course of the infection, indicating insufficient clinical effect. There is also the possibility that viruses that resist sialidase inhibitors may appear. Thus, there is a need for new anti-influenza drugs.

While the inventors of the present invention also reported that F36 (5,7,4'-trihydroxy-8-methoxyflavone), a kind of flavonoid, has anti-influenza virus activity *in vitro* and *in vivo* (T. Nagai, Y Miyaichi, T. Tomimori, Y Suzuki, and H. Yamada, Chem. Pharm. Bull., 38: 1392-1332 (1990); T. Nagai, Y Miyaichi, T. Tomimori, Y. Suzuki, and H. Yamada, Antiviral Res., 19: 207-217 (1992); Japanese Patent No. 2974370), the development of more powerful anti-influenza virus compounds is desired.

Robustaflavone and amentoflavone, which are kinds of biflavones, show anti-influenza virus activities *in vitro,* and the former has also been shown to have anti-influenza virus activity using an *in vivo* mouse system (Japanese Patent Kohyo Publication No. (JP-A) H11-508264 (unexamined Japanese national phase publication corresponding to a non-Japanese international publication), WO97/00679). However, the organic synthesis of biflavonoid-sialic acid conjugates and their anti-influenza virus activities have not been investigated.

Thus, an object of the present invention is to provide new antiviral compounds useful for preventing or treating viral diseases, particularly influenza virus diseases.

### Disclosure of the Invention

The present inventors made extensive investigations to develop anti-influenza virus compounds, and found that various new biflavonoid-sialic acid conjugates have anti-influenza virus activities, thus completing the present invention.

Specifically, the present invention is as follows, for example:

The compounds of the present invention are biflavonoid-sialic acid conjugates.

The aforementioned biflavonoid-sialic acid conjugates may preferably be any compound of general formulae (I), (II), or (III), or salts thereof: 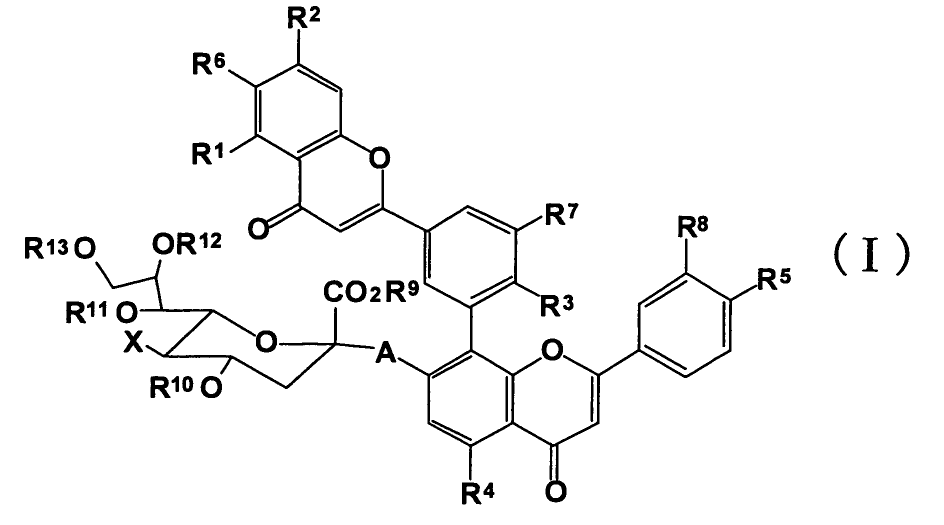 (where in formula (I), R¹, R², R³, R⁴, R⁵, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group; R⁹ is a hydrogen atom, sodium atom, potassium atom, ammonium group, or methyl group; R¹⁰, R¹¹, R¹², and R¹³ are each independently a hydrogen atom, sulfate group, or acetyl group; X is an acetamino group, glycolylamino group, hydroxy group, or acetyloxy group; and A is an oxygen atom or sulfur atom); 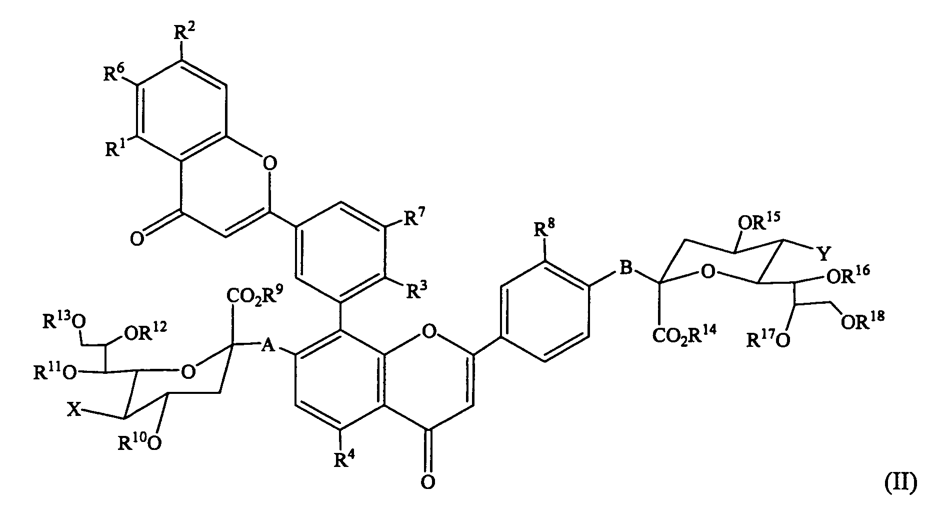 (where in formula (II), R¹, R², R³, R⁴, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group; R⁹ and R¹⁴ are each independently a hydrogen atom, sodium atom, potassium atom, ammonium group, or methyl group; R¹⁰, R¹¹, R¹² ,R¹³, R¹⁵, R¹⁶, R¹⁷, and R¹⁸ are each independently a hydrogen atom, sulfate group, or acetyl group; X and Y are each independently an acetamino group, glycolylamino group, hydroxy group, or acetyloxy group; and A and B are each independently an oxygen atom or sulfur atom); and 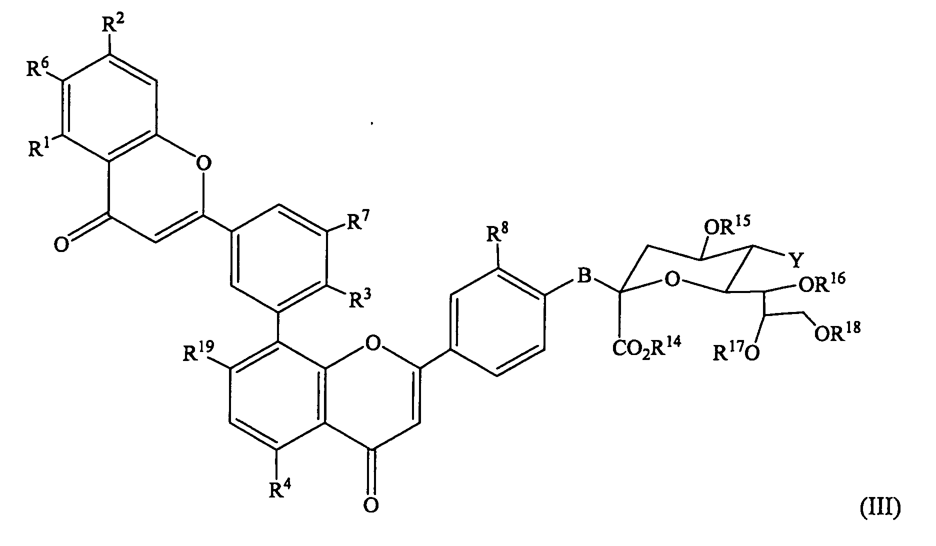 (where in formula (III), R¹, R², R³, R⁴, R⁶, R⁷, R⁸, and R¹⁹ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group; R¹⁴ is a hydrogen atom, sodium atom, potassium atom, ammonium group, or methyl group; R¹⁵, R¹⁶ , R¹⁷ , and R¹⁸ are each independently a hydrogen atom, sulfate group, or acetyl group; Y is an acetamino group, glycolylamino group, hydroxy group, or acetyloxy group; and B is an oxygen atom or sulfur atom).

In the above formulae (I) to (III), R⁹ and R¹⁴ are preferably hydrogen atoms, and R¹⁰, R¹¹, R¹², R¹³, R¹⁵, R¹⁶, R¹⁷, and R¹⁸ are preferably hydrogen atoms.

The pharmaceutical agents of the present invention have the above biflavonoid-sialic acid conjugates as active ingredients.

The preventive or therapeutic agents for influenza of the present invention have the above biflavonoid-sialic acid conjugates as active ingredients.

The food and drink products of the present invention have the above biflavonoid-sialic acid conjugates as active ingredients.

The food and drink products for preventing or treating influenza have the above biflavonoid-sialic acid conjugates as active ingredients.

In addition, the present invention includes methods for preventing or treating influenza which comprise administering effective amounts of the above biflavonoid-sialic acid conjugates.

Moreover, the present invention includes uses of the above biflavonoid-sialic acid conjugates for producing preventive or therapeutic agents for influenza.

In addition, the present invention includes methods of eating and drinking to prevent or treat influenza, which comprise eating and drinking effective amounts of the above biflavonoid-sialic acid conjugates.

Furthermore, the present invention includes uses of the above biflavonoid-sialic acid conjugates for producing food and drink products for preventing or treating influenza.

### Brief Description of the Drawings

Fig. 1 shows a method for purifying biflavonoids from *Cephalotaxus drupacea* Sieb. et Zucc..
Fig. 2 shows a method for purifying biflavonoids from *Ginkgo biloba* L..
Fig. 3 shows a method for synthesizing sugar donors.
Fig. 4 shows a method for synthesizing conjugates of sialic acid derivatives and biflavonoid derivatives.
Fig. 5 shows CD spectra of compounds **29a** and **29b.**
Fig. 6 shows CD spectra of compounds **30a** and **30b.**
Fig. 7 shows CD spectra of compounds **31a** and **31b.**
Fig. 8 shows CD spectra of compounds **32a** and **32b**
Fig. 9 shows structures of the synthesized conjugates of sialic acid derivatives and biflavonoid derivatives.
Fig. 10 shows inhibitory activities against the influenza A/PR/8/34 virus sialidase.
Fig. 11 shows inhibitory activities against the influenza A/Guizhou/54/89 virus sialidase.
Fig. 12 shows inhibitory activities against the influenza B/Ibaraki/2/85 virus sialidase.
Fig. 13 shows effects on the proliferation of influenza virus in MDCK cells.
Fig. 14 shows effects on the survival rate and survival time of mice infected with influenza virus
Fig. 15 shows effects on the mean survival time of mice infected with influenza virus.

### Best Mode for Carrying Out the Invention

The biflavonoid-sialic acid conjugates of the present invention are compounds comprising a biflavonoid-derived component unit and a sialic acid-derived component unit.

The term biflavonoid-derived component unit means a component unit derived from a compound in which two flavonoid backbones have been connected, and the term sialic acid backbone means a component unit derived from neuraminic acid derivatives, which are amino sugars having 9 carbon atoms, or KDN (2-keto-3-deoxy-D-glycero-D-galacto-2-nonulosonic acid) derivatives.

The biflavonoid-sialic acid conjugates of the present invention comprise at least one sialic acid-derived component unit, and preferably comprise one or two sialic acid-derived component units.

More specifically, such biflavonoid-sialic acid conjugates include, for example, any compound shown below by general formula (I), (II), or (III), or salts thereof.

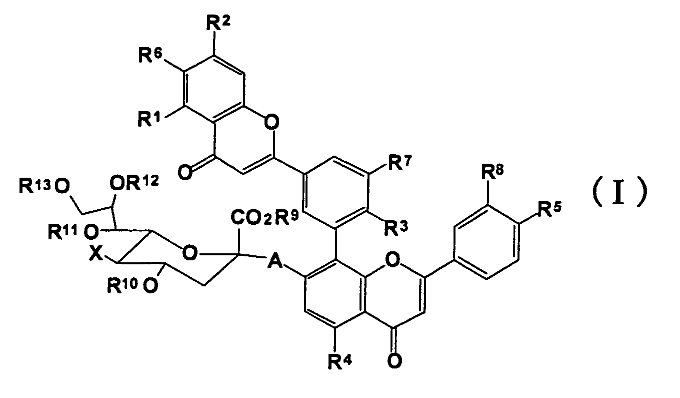

In formula (I), R¹, R², R³, R⁴, R⁵, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group.

Of these, R¹, R², R³, R⁴, and R⁵ are preferably a hydroxy group, acetyloxy group, methyloxy group, ethyloxy group, n-propyloxy group, n-butyloxy group, n-octyloxy group, benzyloxy group, or allyloxy group, and more preferably hydroxy group or methyloxy group. Moreover, at least one, and more preferably one to three of R¹, R², R³, R⁴, and R⁵ may be a hydroxy group.

More specifically, combinations of R¹, R², R³, R⁴, and R⁵ include those where, for example:
R¹ is a hydroxy group, R² is a methyloxy group, R³is a methyloxy group, R⁴ is a hydroxy group, and R⁵ is a hydroxy group;
R¹ is a hydroxy group, R² is a methyloxy group, R³ is a methyloxy group, R⁴ is a hydroxy group, and R⁵ is a methyloxy group;
R¹ is a hydroxy group, R² is a hydroxy group, R³ is a methyloxy group, R⁴ is a hydroxy group, and R⁵ is a methyloxy group;
R¹ is a hydroxy group, R² is a hydroxy group, R³ is a methyloxy group, R⁴ is a hydroxy group, and R⁵ is a hydroxy group;
R¹ is a hydroxy group, R² is a hydroxy group, R³ is a hydroxy group, R⁴ is a hydroxy group, and R⁵ is a hydroxy group;
R¹ is a hydroxy group, R² is a methyloxy group, R³ is a hydroxy group, R⁴ is a hydroxy group, and R⁵ is hydroxy group;
R¹ is a hydroxy group, R² is a hydroxy group, R³ is a hydroxy group, R⁴ is a hydroxy group, and R⁵ is methyloxy group; and
R¹ is a hydroxy group, R² is a methyloxy group, R³ is a hydroxy group, R⁴ is a hydroxy group, and R⁵ is a methyloxy group.

Of these, preferably, R¹ is a hydroxy group, R² is a methyloxy group, R³ is a methyloxy group, R⁴ is a hydroxy group, and R⁵ is a hydroxy group; or

R¹ is a hydroxy group, R² is a methyloxy group, R³ is a methyloxy group, R⁴ is a hydroxy group, and R⁵ is a methyloxy group.

R⁶, R⁷, and R⁸ may preferably be a hydrogen atom, hydroxy group, methyl group, or methyloxy group, and more preferably a hydrogen atom.

More specifically, combinations of R⁶, R⁷, and R⁸ include those where, for example:
R⁶ is a hydrogen atom, R⁷ is a hydrogen atom, and R⁸ is a hydrogen atom;
R⁶ is a hydrogen atom, R⁷ is a hydroxy group, and R⁸ is a hydrogen atom;
R⁶ is a hydrogen atom, R⁷ is a methyloxy group, and R⁸ is a hydrogen atom;
R⁶ is a hydrogen atom, R⁷ is a hydrogen atom, and R⁸ is a hydroxy group;
R⁶ is a hydrogen atom, R⁷ is a hydroxy group, and R⁸ is a hydroxy group; and
R⁶ is a methyl group, R⁷ is a hydrogen atom, and R⁸ is a hydrogen atom.

R⁹ is a hydrogen atom, sodium atom, potassium atom, ammonium group, or methyl group, and preferably a hydrogen atom.

R¹⁰, R¹¹, R¹², and R¹³ are each independently a hydrogen atom, sulfate group, or acetyl group. Preferably, R¹⁰, R¹¹, R¹², and R¹³ are each independently a hydrogen atom or acetyl group, and more preferably a hydrogen atom.

Herein, sulfate group refers to a group expressed by HO₃S- or salts thereof, and acetyl group refers to a group expressed by CH₃CO-.

Moreover, most preferably, R⁹, R¹⁰, R¹¹, R¹², and R¹³ are each hydrogen atoms.

Thus, the inhibitory activity against influenza virus sialidase and the anti-influenza virus activity of the biflavonoid-sialic acid conjugates shown by the above formula (I) will increase when R⁹, R¹⁰, R¹¹, R¹² , and R¹³ are each hydrogen atoms.

X represents an acetamino group, glycolylamino group, hydroxy group, or acetyloxy group, and preferably an acetamino group or glycolylamino group.

Herein, an acetamino group represents a group expressed by CH₃CONH-; a glycolylamino group represents a group expressed by HOCH₂CH(OH)NH-; and an acetyloxy group represents a group expressed by CH₃COO-.

A represents an oxygen atom or sulfur atom, and preferably an oxygen atom.

Specific examples of the biflavonoid-sialic acid conjugates shown by the above formula (I) include the compounds below, for example:
biflavonoid-sialic acid conjugate (34), wherein R¹=hydroxy group, R²=methyloxy group, R³=methyloxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹² =hydrogen atom, R¹³=hydrogen atom, A=oxygen atom, and X=acetamino group (NHAc) (the number in parenthesis following the compound represents the of the compound number in the Examples. The same goes for the compounds hereinafter.);
biflavonoid-sialic acid conjugate (36), wherein R¹=hydroxy group, R²=methyloxy group, R³=methyloxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹² =hydrogen atom, R¹³=hydrogen atom, A=oxygen atom, and X=acetamino group;
biflavonoid-sialic acid conjugate, wherein R¹=hydroxy group, R²=hydroxy group, R³=methyloxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=hydroxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²= hydrogen atom, R¹³=hydrogen atom, A=oxygen atom, and X=acetamino group;
biflavonoid-sialic acid conjugate, wherein R¹=hydroxy group, R²=methyloxy group, R³=hydroxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=methyloxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=hydroxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=methyloxy group, R³=hydroxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=hydroxy group, R⁴=hydroxy group, R⁵=hydroxy group, R⁶=hydrogen atom, R⁷=hydroxy group, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=methyloxy group, R⁴=hydroxy group, R⁵=hydroxy group, R⁶=hydrogen atom, R⁷=methyloxy group, R⁸=hydrogen atom, R⁹=hydrogen atom, R¹⁰=hydrogen atom, R¹¹=hydrogen atom, R¹² =hydrogen atom, R¹³=hydrogen atom, A=oxygen atom, and X=acetamino group;
biflavonoid-sialic acid conjugate, wherein R¹=hydroxy group, R²=hydroxy group, R³=hydroxy group, R⁴=hydroxy group, R⁵=hydroxy group, R⁶=hydrogen atom, R⁷=hydrogen atom, R⁸=hydroxy group, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=hydroxy group, R³=hydroxy group, R⁴=hydroxy group, R⁵=hydroxy group, R⁶=hydrogen atom, R⁷=hydroxy group, R⁸=hydroxy group, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=methyloxy group, R³=hydroxy group, R⁴=hydroxy group, R⁵=hydroxy group, R⁶=methyl group, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=acetamino group;
biflavonoid-sialic acid conjugate, wherein R¹=hydroxy group, R²=methyloxy group, R³=methyloxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X= glycolylamino group;
biflavonoid-sialic acid conjugate, wherein R¹=hydroxy group, R²=methyloxy group, R³=methyloxy group, R⁴=hydroxy group, R⁵=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=hydroxy group;
biflavonoid-sialic acid conjugate, wherein R¹=hydroxy group, R²=methyloxy group, R³=methyloxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=glycolyloxy group; and
biflavonoid-sialic acid conjugate, wherein R¹=hydroxy group, R²=methyloxy group, R³=methyloxy group, R⁴=hydroxy group, R⁵=methyloxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, A=oxygen atom, and X=hydroxy group.

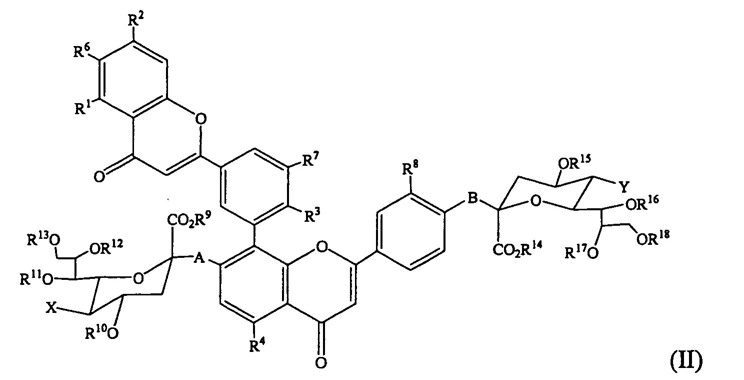

In formula (II), R¹, R², R³, R⁴, R⁵, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group.

Of these, R¹, R², R³, and R⁴ are each preferably a hydroxy group, acetyloxy group, methyloxy group, ethyloxy group, n-propyloxy group, n-butyloxy group, n-octyloxy group, benzyloxy group, or allyloxy group, and more preferably a hydroxy group or methyloxy group. Moreover, at least one, and more preferably one to three of R¹, R², R³, and R⁴ are each a hydroxy group.

More specifically, combinations of R¹, R², R³, and R⁴ include those where, for example:
R¹ is a hydroxy group, R² is a methyloxy group, R³ is a methyloxy group, and R⁴ is a hydroxy group;
R¹ is a hydroxy group, R² is a hydroxy group, R³ is a methyloxy group, and R⁴ is a hydroxy group;
R¹ is a hydroxy group, R² is a hydroxy group, R³ is a hydroxy group, and R⁴ is a hydroxy group; and
R¹ is a hydroxy group, R² is a methyloxy group, R³ is a hydroxy group, and R⁴ is a hydroxy group.

Of these, preferably, R¹ is a hydroxy group, R² is a methyloxy group, R³ is a methyloxy group, and R⁴ is a hydroxy group.

R⁶, R⁷, and R⁸ are each preferably a hydrogen atom, hydroxy group, methyl group, or methyloxy group, and more preferably a hydrogen atom.

More specifically, combinations of R⁶, R⁷, and R⁸ include those where, for example:
R⁶ is a hydrogen atom, R⁷ is a hydrogen atom, and R⁸ is a hydrogen atom;
R⁶ is a hydrogen atom, R⁷ is a hydroxy group, and R⁸ is a hydrogen atom;
R⁶ is a hydrogen atom, R⁷ is a methyloxy group, and R⁸ is a hydrogen atom;
R⁶ is a hydrogen atom, R⁷ is a hydrogen atom, and R⁸ is a hydroxy group;
R⁶ is a hydrogen atom, R⁷ is a hydroxy group, and R⁸ is a hydroxy group; and
R⁶ is a methyl group, R⁷ is a hydrogen atom, and R⁸ is a hydrogen atom.

R⁹ and R¹⁴ are each independently a hydrogen atom, sodium atom, potassium atom, ammonium group, or methyl group, and preferably a hydrogen atom.

R¹⁰, R¹¹, R¹², R¹³ R¹⁵, R¹⁶, R¹⁷ and R¹⁸ are each independently a hydrogen atom, sulfate group, or acetyl group. Preferably, R¹⁰, R¹¹, R¹², R¹³, R¹⁵, R¹⁶, R¹⁷ and R¹⁸ are each independently a hydrogen atom or acetyl group, and more preferably a hydrogen atom.

Moreover, most preferably, R⁹, R¹⁴, R¹⁰, R¹¹, R¹², R¹³, R¹⁵, R¹⁶, R¹⁷ and R¹⁸ are each hydrogen atoms.

Thus, the inhibitory activity against influenza virus sialidase and the anti-influenza virus activity of the biflavonoid-sialic acid conjugates of the above formula (II) will increase when R⁹ and R¹⁴ are each hydrogen atoms, and R¹⁰, R¹¹, R¹², R¹³, R¹⁵, R¹⁶, R¹⁷ and R¹⁸ are each hydrogen atoms.

X and Y are each independently an acetamino group, glycolylamino group, hydroxy group, or acetyloxy group, and preferably an acetamino group or glycolylamino group.

A and B are each independently an oxygen atom or sulfur atom, and preferably an oxygen atom.

Specific examples of the biflavonoid-sialic acid conjugates shown by the above formula (II) include the compounds below, for example:
biflavonoid-sialic acid conjugate (35), wherein R¹=hydroxy group, R²=methyloxy group, R³=methyloxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸ =hydrogen atom, A=B=oxygen atom, and X=Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸⁼hydrogen atom, A=B=oxygen atom, and X=Y=acetamino group;
biflavonoid-sialic acid conjugate, wherein R¹=hydroxy group, R²=methyloxy group, R³=hydroxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸=hydrogen atom, A=B=oxygen atom, and X=Y=acetamino group;
biflavonoid-sialic acid conjugate, wherein R¹=hydroxy group, R²=hydroxy group, R³=methyloxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³ =hydrogen atom, R¹⁴=hydrogen atom, R¹⁵=hydrogen atom, R¹⁶=hydrogen atom, R¹⁷=hydrogen atom, R¹⁸ =hydrogen atom, A=B=oxygen atom, and X=Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydroxy group, R⁸=hydrogen atom, R⁹=hydrogen atom, R¹⁰=hydrogen atom, R¹¹=hydrogen atom, R¹²=hydrogen atom, R¹³ =hydrogen atom, R¹⁴=hydrogen atom, R¹⁵=hydrogen atom, R¹⁶=hydrogen atom, R¹⁷ =hydrogen atom, R¹⁸ =hydrogen atom, A=B=oxygen atom, and X=Y=acetamino group;
biflavonoid-sialic acid conjugate, wherein R¹=hydroxy group, R²=hydroxy group, R³=methyloxy group, R⁴=hydroxy group, R⁶=hydrogen atom, R⁷=methyloxy group, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵ =hydrogen atom, R¹⁶=hydrogen atom, R¹⁷=hydrogen atom, R¹⁸=hydrogen atom, A=B=oxygen atom, and X=Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydrogen atom, R⁸=hydroxy group, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵ =hydrogen atom, R¹⁶=hydrogen atom, R¹⁷=hydrogen atom, R¹⁸=hydrogen atom, A=B=oxygen atom, and X=Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydroxy group, R⁸=hydroxy group, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷ =hydrogen atom, R¹⁸=hydrogen atom, A=B=oxygen atom, and X=Y=acetamino group;
biflavonoid-sialic acid conjugate, wherein R¹=hydroxy group, R²=methyloxy group, R³=hydroxy group, R⁴=hydroxy group, R⁶=methyl group, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸=hydrogen atom, A=B=oxygen atom, and X=Y=acetamino group;
biflavonoid-sialic acid conjugate, wherein R¹=hydroxy group, R²=methyloxy group, R³=methyloxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸=hydrogen atom, A=B=oxygen atom, and X=Y= glycolylamino group; and
biflavonoid-sialic acid conjugate, wherein R¹=hydroxy group, R²=methyloxy group, R³=methyloxy group, R⁴=hydroxy group, R⁶=hydrogen atom, R⁷=hydrogen atom, R⁸=hydrogen atom, R⁹=hydrogen atom, R¹⁰=hydrogen atom, R¹¹=hydrogen atom, R¹²=hydrogen atom, R¹³=hydrogen atom, R¹⁴=hydrogen atom, R¹⁵=hydrogen atom, R¹⁶=hydrogen atom, R¹⁷=hydrogen atom, R¹⁸=hydrogen atom, A=B=oxygen atom, and X=Y=hydroxy group.

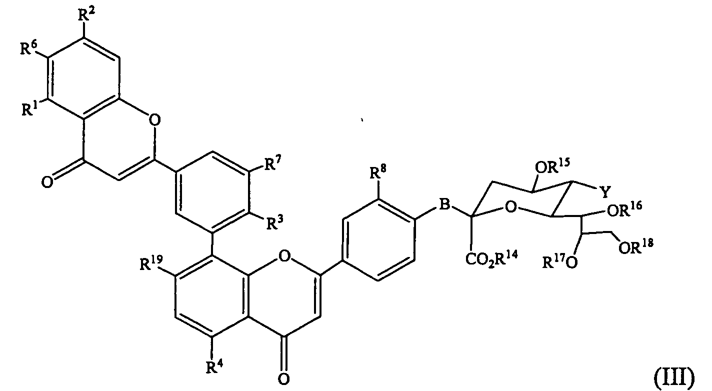

In formula (III), R¹, R², R³, R⁴, R⁶, R⁷, R⁸ and R¹⁹ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group.

Of these, R¹, R², R³, R⁴, and R¹⁹ are each preferably a hydroxy group, acetyloxy group, methyloxy group, ethyloxy group, n-propyloxy group, n-butyloxy group, n-octyloxy group, benzyloxy group, or allyloxy group, and more preferably a hydroxy group or methyloxy group. Moreover, at least one, and more preferably one to three of R¹, R², R³, R⁴ and R¹⁹ may be hydroxy groups.

More specifically, combinations of R¹, R², R³, R⁴, and R¹⁹ include those where, for example:
R¹ is a hydroxy group, R² is a methyloxy group, R³ is a methyloxy group, R⁴ is a hydroxy group, and R¹⁹ is a methyloxy group;
R¹ is a hydroxy group, R² is a hydroxy group, R³ is a methyloxy group, R⁴ is a hydroxy group, and R¹⁹ is a methyloxy group;
R¹ is a hydroxy group, R² is a hydroxy group, R³ is a hydroxy group, R⁴ is a hydroxy group, and R¹⁹ is a hydroxy group;
R¹ is a hydroxy group, R² is a methyloxy group, R³ is a hydroxy group, R⁴ is a hydroxy group, and R¹⁹ is a hydroxy group;
R¹ is a hydroxy group, R² is a hydroxy group, R³ is a hydroxy group, R⁴ is a hydroxy group, and R¹⁹ is a methyloxy group;
R¹ is a hydroxy group, R² is a methyloxy group, R³ is a hydroxy group, R⁴ is a hydroxy group, and R¹⁹ is a methyloxy group;
R¹ is a hydroxy group, R² is a hydroxy group, R³ is a methyloxy group, R⁴ is a hydroxy group, and R¹⁹ is a hydroxy group; and
R¹ is a hydroxy group, R² is a methyloxy group, R³ is a methyloxy group, R⁴ is a hydroxy group, and R¹⁹ is a hydroxy group. Of these, preferably, R¹ is a hydroxy group, R² is a methyloxy group, R³ is a methyloxy group, R⁴ is a hydroxy group, and R¹⁹ is a methyloxy group.

R⁶, R⁷, and R⁸ may preferably be a hydrogen atom, hydroxy group, methyl group, or methyloxy group, and more preferably a hydrogen atom.

More specifically, combinations of R⁶, R⁷, and R⁸ include those where, for example:
R⁶ is a hydrogen atom, R⁷ is a hydrogen atom, and R⁸ is a hydrogen atom;
R⁶ is a hydrogen atom, R⁷ is a hydroxy group, and R⁸ is a hydrogen atom;
R⁶ is a hydrogen atom, R⁷ is a methyloxy group, and R⁸ is a hydrogen atom;
R⁶ is a hydrogen atom, R⁷ is a hydrogen atom, and R⁸ is a hydroxy group;
R⁶ is a hydrogen atom, R⁷ is a hydroxy group, and R⁸ is a hydroxy group; and
R⁶ is a methyl group, R⁷ is a hydrogen atom, and R⁸ is a hydrogen atom.

R¹⁴ represents a hydrogen atom, sodium atom, potassium atom, ammonium group, or methyl group, and preferably a hydrogen atom.

R¹⁵, R¹⁶, R¹⁷ and R¹⁸ are each independently a hydrogen atom, sulfate group, or acetyl group. Preferably, R¹⁵, R¹⁶, R¹⁷ and R¹⁸ are each independently a hydrogen atom or acetyl group, and more preferably a hydrogen atom.

Moreover, most preferably, R¹⁴, R¹⁵, R¹⁶, R¹⁷ and R¹⁸ are each hydrogen atoms.

Thus, the inhibitory activity against influenza virus sialidase and the anti-influenza virus activity of the biflavonoid-sialic acid conjugates shown by the above formula (III) will increase when R¹⁴ is a hydrogen atom, and R¹⁵, R¹⁶, R¹⁷ and R¹⁸ are each hydrogen atoms.

Y represents an acetamino group, glycolylamino group, hydroxy group, or acetyloxy group, and preferably an acetamino group or glycolylamino group.

B represents an oxygen atom or sulfur atom, and preferably an oxygen atom.

Specific examples of the biflavonoid-sialic acid conjugates shown by the above formula (III) include the compounds below, for example:
biflavonoid-sialic acid conjugate (33), wherein R¹=hydroxy group, R²=methyloxy group, R³=methyloxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷ =hydrogen atom, R¹⁸=hydrogen atom, R¹⁹=methyloxy group, B=oxygen atom, and Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=methyloxy group, R³=hydroxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸ =hydrogen atom, R¹⁹=hydroxy group, B=oxygen atom, and Y=acetamino group;
biflavonoid-sialic acid conjugate, wherein R¹=hydroxy group, R²=hydroxy group, R³=methyloxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=methyloxy group, B=oxygen atom, and Y=acetamino group;
biflavonoid-sialic acid conjugate, wherein R¹=hydroxy group, R²=methyloxy group, R³=methyloxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=methyloxy group, R³=hydroxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=methyloxy group, B=oxygen atom, and Y=acetamino group;
biflavonoid-sialic acid conjugate, wherein R¹=hydroxy group, R²=hydroxy group, R³=methyloxy group, R⁴=hydroxy group, R⁶=hydrogen atom, R⁷=methyloxy group, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=methyloxy group, B=oxygen atom, and Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydroxy group, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=hydroxy group, R³=methyloxy group, R⁴=hydroxy group, R⁶=hydrogen atom, R⁷=methyloxy group, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydrogen atom, R⁸=hydroxy group, R¹⁴=hydrogen atom, R¹⁵=hydrogen atom, R¹⁶=hydrogen atom, R¹⁷ =hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=hydroxy group, R³=hydroxy group, R⁴=hydroxy group, R⁶=hydrogen atom, R⁷=hydroxy group, R⁸=hydroxy group, R¹⁴=hydrogen atom, R¹⁵=hydrogen atom, R¹⁶=hydrogen atom, R¹⁷ =hydrogen atom, R¹⁸ =hydrogen atom, R¹⁹=hydroxy group, B=oxygen atom, and Y=acetamino group;
biflavonoid-sialic acid conjugate, wherein R¹=hydroxy group, R²=methyloxy group, R³=hydroxy group, R⁴=hydroxy group, R⁶=methyl group, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=hydroxy group, B=oxygen atom, and Y=acetamino group;
biflavonoid-sialic acid conjugate, wherein R¹=hydroxy group, R²=methyloxy group, R³=methyloxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸ =hydrogen atom, R¹⁹=methyloxy group, B=oxygen atom, and Y= glycolylamino group; and
biflavonoid-sialic acid conjugate, wherein R¹=hydroxy group, R²=methyloxy group, R³=methyloxy group, R⁴=hydroxy group, R⁶=hydrogen atom, R⁷=hydrogen atom, R⁸=hydrogen atom, R¹⁴=hydrogen atom, R¹⁵=hydrogen atom, R¹⁶=hydrogen atom, R¹⁷=hydrogen atom, R¹⁸=hydrogen atom, R¹⁹=methyloxy group, B=oxygen atom, and Y= hydroxy group.

These biflavonoid-sialic acid conjugates shown by the above formulae (I) to (III) may form salts depending on the type of substitutive groups. Pharmaceutically acceptable salts of the compounds shown by the above formulae (I) to (III) may also be used for the present invention's pharmaceutical agents, or preventive or therapeutic agents for influenza, as well as food and drink products or food and drink products for preventing or treating influenza, as will be described later.

Such salts of the compounds shown by the above formulae (I) to (III) include, for example, metal salts such as sodium salts, potassium salts, or calcium salts, organic ammonium salts such as ammonium salts, and amino acid salts such as glycine salts. In addition to free forms of the compounds or salts thereof, any hydrate form thereof may also be used. The salts can be formed using standard procedures.

For example, R⁹ and R¹⁴ may each independently be sodium or potassium to form metal salts, or ammonium to form organic ammonium salts.

While the compounds shown by the above formulae (I) to (III) may preferably have the conformation indicated in the formulae, they may exist as optically active compounds due to their asymmetric carbons. The present invention includes such stereoisomers as optically active isomers and diastereomers, any mixture of stereoisomers, or racemic compounds. In addition, in the above formulae (I) to (III), the configuration of CO₂R⁹ and A that connect to the asymmetric carbon show a relative configuration, not an absolute configuration. This is also true of the configurations of the other asymmetric carbons.

These biflavonoid-sialic acid conjugates, in which a constitutional unit from biflavonoid and a constitutional unit from sialic acid have been connected, may be produced, for example, by coupling a biflavonoid derivative with a sialic acid derivative.

Methods for the production of such sialic acid derivatives used for the coupling reaction will be explained using, for example, sialic acid derivative (IV-1) shown by formula (IV) below as a starting material, in which R⁹, R¹⁰, R¹¹, R¹², and R¹³ are hydrogen atoms, X is an acetamino group, glycolylamino group, or hydroxy group, and L is a hydroxy group.

First of all, among the compounds of formula (IV): 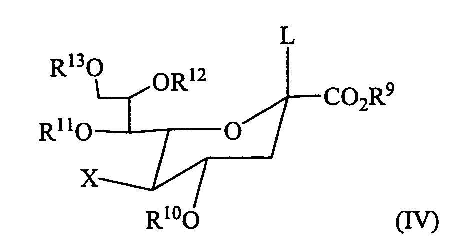 (which hereinafter may also be called "sialic acid derivative (IV)), sialic acid derivative (IV-1) which is commercially available, for example, in which R⁹, R¹⁰, R¹¹, R¹², and R¹³ are hydrogen atoms, L is a hydroxy group, X is an acetamino group, glycolylamino group, or hydroxy group, and L is a hydroxy group, may be esterified in the presence of alcohol, for example (R⁹ is converted from H to CH₃ by esterification with methanol).

Any known method may be used for the esterification reaction, without limitation. For example, the esterification reaction may be carried out by dissolving sialic acid derivative (IV-1) in an excess of alcohol, such as methanol, and stirring generally in the presence of a dehydrating agent at or around room temperature for about 5 to 100 hours.

In this way, sialic acid derivative (IV-2), in which a carboxyl group is esterified, may be obtained.

Next, hydroxy groups of the obtained esterified sialic acid derivative (IV-2) are protected with acetyl groups. Acetylation of the hydroxy groups may be carried out according to known method. For example, sialic acid derivative (IV-2) is reacted with an acetylation reagent in a solvent such as pyridine, optionally in the presence of a catalyst. The acetylation reagent includes acetic anhydride and acetyl chloride. The catalysts include 4-dimethylaminopyridine (DMAP) and the like. The reaction may be carried out preferably by using 1.2 to 3 equivalents of acetylation reagent per hydroxy group and stirring at 0 to 30°C, generally at or around room temperature for about 5 to 100 hours. In this way, sialic acid derivative (IV-3), in which a hydroxy group is acetylated, can be obtained.

Moreover, sialic acid derivative (IV-3) may be reacted with, for example, a halogenating reagent in the presence of a catalyst, to yield sialic acid derivative (IV-4), in which L is halogenated.

The halogenating reagents include acetyl halides such as acetyl chloride. The catalysts include hydrogen chloride gas and sulfuric acid. The reaction may be carried out by, for example, dissolving sialic acid derivative (IV-3) in excess equivalents of a halogenating reagent, adding a catalyst such as hydrogen chloride gas under the conditions of temperature at -10°C to room temperature, and stirring for about 5 to 100 hours.

In this way, sialic acid derivative (IV-4) of the above formula (IV), which can be used for the coupling reaction, may be obtained such that R¹⁰, R¹¹, R¹², and R¹³ are acetyl groups, R⁹ is a methyl group, L is a halogen atom, X is an acetamino group, glycolylamino group, or acetyloxy group.

Biflavonoid derivatives of formula (V) (which hereinafter may also be called "biflavonoid derivative (V)") may be used for the production of the biflavonoid-sialic acid conjugates of the present invention.

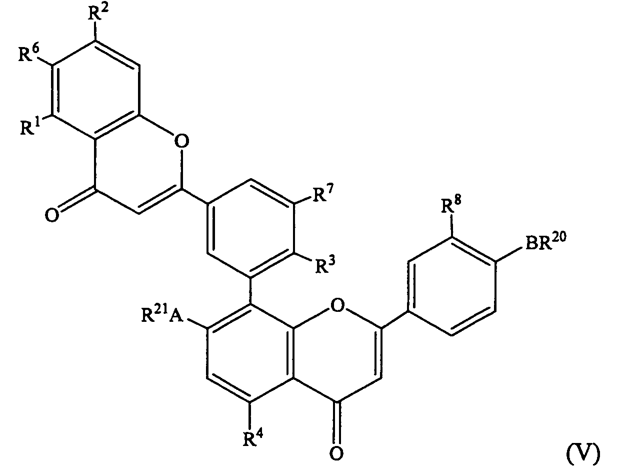

In biflavonoid derivative (V) of the above formula, R¹, R², R³, R⁴, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, or allyloxy group.

R²⁰ and R²¹ are each independently a hydrogen atom, methyl group, acetyl group, ethyl group, n-propyl group, n-butyl group, n-octyl group, benzyl group, allyl group, or glycosyl group, and at least one of R²⁰ and R²¹ is a hydrogen atom.

A and B each independently represent an oxygen atom or sulfur atom.

This biflavonoid derivative (V) may be purified from commercially available products or natural products.

In addition, functional groups of biflavonoid derivative (V) isolated from a commercially available product or natural source may be converted by standard methods to yield biflavonoid derivative having a desired functional group.

The biflavonoid derivatives available as naturally occurring substances or commercially available products include, for example, the following compounds:
biflavonoid derivative (19), wherein R¹=hydroxy group, R²=methyloxy group, R³=methyloxy group, R⁴=hydroxy group, R⁶=hydrogen atom, R⁷=hydrogen atom, R⁸=hydrogen atom, R²⁰=hydrogen atom, R²¹=methyl group, and A=B=oxygen atom;
biflavonoid derivative (20), wherein R¹=hydroxy group, R²=methyloxy group, R³=methyloxy group, R⁴=hydroxy group, R⁶=hydrogen atom, R⁷=hydrogen atom, R⁸=hydrogen atom, R²⁰=hydrogen atom, R²¹=hydrogen atom, and A=B=oxygen atom;
biflavonoid derivative (21), wherein R¹=hydroxy group, R²=methyloxy group, R³=methyloxy group, R⁴=hydroxy group, R⁶=hydrogen atom, R⁷=hydrogen atom, R⁸=hydrogen atom, R²⁰=methyl group, R²¹=hydrogen atom, and A=B=oxygen atom;
biflavonoid derivative (22), wherein R¹=hydroxy group, R²=hydroxy group, R³=methyloxy group, R⁴=hydroxy group, R⁶=hydrogen atom, R⁷=hydrogen atom, R⁸=hydrogen atom, R²⁰=methyl group, R²¹=hydrogen atom, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydrogen atom, R⁸=hydrogen atom, R²⁰=hydrogen atom, R²¹=hydrogen atom, and A=B=oxygen atom;
biflavonoid derivative, wherein R¹=hydroxy group, R²=methyloxy group, R³=hydroxy group, R⁴=hydroxy group, R⁶=hydrogen atom, R⁷=hydrogen atom, R⁸=hydrogen atom, R²⁰=hydrogen atom, R²¹=hydrogen atom, and A=B=oxygen atom;
biflavonoid derivative, wherein R¹=hydroxy group, R²=hydroxy group, R³=methyloxy group, R⁴=hydroxy group, R⁶=hydrogen atom, R⁷=hydrogen atom, R⁸=hydrogen atom, R²⁰=hydrogen atom, R²¹=hydrogen atom, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydrogen atom, R⁸=hydrogen atom, R²⁰=methyl group, R²¹=hydrogen atom, and A=B=oxygen atom;
biflavonoid derivative, wherein R¹=hydroxy group, R²=methyloxy group, R³=hydroxy group, R⁴=hydroxy group, R⁶=hydrogen atom, R⁷=hydrogen atom, R⁸=hydrogen atom, R²⁰=methyl group, R²¹=hydrogen atom, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydrogen atom, R⁸=hydrogen atom, R²⁰=hydrogen atom, R²¹=methyl group, and A=B=oxygen atom;
biflavonoid derivative, wherein R¹=hydroxy group, R²=methyloxy group, R³=hydroxy group, R⁴=hydroxy group, R⁶=hydrogen atom, R⁷=hydrogen atom, R⁸=hydrogen atom, R²⁰=hydrogen atom, R²¹=methyl group, and A=B=oxygen atom;
biflavonoid derivative, wherein R¹=hydroxy group, R²=hydroxy group, R³=methyloxy group, R⁴=hydroxy group, R⁶=hydrogen atom, R⁷=hydrogen atom, R⁸=hydrogen atom, R²⁰=hydrogen atom, R²¹=methyl group, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydroxy group, R⁸=hydrogen atom, R²⁰=hydrogen atom, R²¹=hydrogen atom, and A=B=oxygen atom;
biflavonoid derivative, wherein R¹=hydroxy group, R²=hydroxy group, R³=methyloxy group, R⁴=hydroxy group, R⁶=hydrogen atom, R⁷=methyloxy group, R⁸=hydrogen atom, R²⁰=hydrogen atom, R²¹=hydrogen atom, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydrogen atom, R⁸=hydroxy group, R²⁰=hydrogen atom, R²¹=hydrogen atom, and A=B=oxygen atom;
biflavonoid derivative, wherein R¹=hydroxy group, R²=hydroxy group, R³=hydroxy group, R⁴=hydroxy group, R⁶=hydrogen atom, R⁷=hydroxy group, R⁸=hydroxy group, R²⁰=hydrogen atom, R²¹=hydrogen atom, and A=B=oxygen atom;
biflavonoid derivative, wherein R¹=hydroxy group, R²=methyloxy group, R³=hydroxy group, R⁴=hydroxy group, R⁶=methyl group, R⁷=hydrogen atom, R⁸=hydroxy group, R²⁰=hydrogen atom, R²¹=hydrogen atom, and A=B=oxygen atom;
biflavonoid derivative, wherein R¹=hydroxy group, R²=glucosyloxy group, R³=hydroxy group, R⁴=hydroxy group, R⁶=hydrogen atom, R⁷=hydrogen atom, R⁸=hydrogen atom, R²⁰=glucosyl group, R²¹=hydrogen atom, and A=B=oxygen atom;
biflavonoid derivative, wherein R¹=hydroxy group, R²=hydroxy group, R³=glucosyloxy group, R⁴=hydroxy group, R⁶=hydrogen atom, R⁷=hydrogen atom, R⁸=hydrogen atom, R²⁰=glucosyl group, R²¹=hydrogen atom, and A=B=oxygen atom;
biflavonoid derivative, wherein R¹=hydroxy group, R²=glucosyloxy group, R³=glucosyloxy group, R⁴=hydroxy group, R⁶=hydrogen atom, R⁷=hydrogen atom, R⁸=hydrogen atom, R²⁰=glucosyl group, R²¹=hydrogen atom, and A=B=oxygen atom.

Methods for the production of the biflavonoid-sialic acid conjugates will be explained using the reaction of the biflavonoid derivative (V) thus obtained with the sialic acid derivative (IV-4) mentioned above, as an example.

The reaction of the above-mentioned sialic acid derivative (IV-4) with the above-mentioned biflavonoid derivative (V) may be carried out, for example, by contacting the above-mentioned sialic acid derivative (IV-4) with the above-mentioned biflavonoid derivative (V) in a solvent in the presence of a base.

Examples of the bases include, for example, hydrogenated alkali metals such as NaH and KH. The solvents include amide solvents like N,N-dimethylformamide (DMF), and HMPA.

Though the amount of base depends on the number of sialic acid derivatives to be bound to biflavonoid derivatives, it is preferably within the range of 1.2 to 1.8 times the amount of biflavonoid derivative when one sialic acid derivative is to be bound, for example. When two sialic acid derivatives are bound, the amount of the base is preferably within the range of 2.2 to 3.5 times the amount of biflavonoid derivative.

The amount of a sialic acid derivative to be used is preferably within the range of 1.2 to 1.8 times the amount of a biflavonoid when one sialic acid derivative is bound. When two sialic acid derivatives are bound, it is preferably within the range of 2.2 to 3.5 times the amount of biflavonoid derivative.

The reaction may be carried out by stirring at 0 to 30°C, or generally at or around room temperature, for about 5 to 100 hours.

The above-mentioned biflavonoid-sialic acid conjugates of formulae (I) to (III) in which A and B are sulfur atoms may be obtained by, for example, reacting the sialic acid derivative (IV-5) in which L in the formula (IV) is a sulfur-containing group, with the compound of formula (V) described below, in which at least one of R²¹A- and R²⁰B- is a halogen atom.

Sialic acid derivative (IV-5) in which L is a sulfur-containing group may be obtained, for example, by contacting the sialic acid derivative (IV-4) mentioned above with AcSK (potassium thioacetate).

One compound of biflavonoid derivative (V), in which at least one of R²¹A- and R²⁰Bis a halogen atom, may be obtained by reacting a compound of flavonoid derivative (V), in which at least one of R²¹A- and R²⁰B- is a hydroxy group, with a halogenation reagent such as NBS in a solvent in the presence of triphenyl phosphine or such.

Moreover, the biflavonoid halide thus obtained may be reacted with sialic acid derivative (IV-5) in a solvent such as DMF in the presence of an amine or such, to produce the biflavonoid-sialic acid conjugates of formulae (I) to (III) mentioned above, in which A and B are sulfur atoms.

The resulting biflavonoid-sialic acid conjugates of the present invention are protected sialyl biflavonoids in which the hydroxy group moieties and the carboxyl groups of the sialic acid-derived component have been protected.

Such protected sialyl biflavonoid may subsequently be hydrolyzed using standard methods to deprotect the acetyl groups and also convert the carboxylic acid ester group to the carboxyl group.

Such biflavonoid-sialic acid conjugates (in which, for example, R⁹ and R¹⁴ are hydrogen atoms, and R¹⁰, R¹¹, R¹², R¹³, R¹⁵, R¹⁶, R¹⁷, and R¹⁸ are hydrogen atoms), in which each functional group derived from the sialic acid component has been converted to a hydroxy group or carboxyl group, or salts thereof, has superior inhibitory activity against the influenza virus sialidase and has superior anti-influenza virus activity.

The above-mentioned biflavonoid derivatives obtained as naturally occurring materials and the like, having functional groups such as hydroxy groups, may be derivatized to various derivatives by functional group conversion of hydroxy group moieties and such. They can then be used for coupling with sialic acid derivatives.

For example, each hydroxy group moiety of a biflavonoid derivative (V) that is used as a raw material for coupling may be alkyl-etherified using standard methods. The alkyletherification may be carried out, for example, by reacting the biflavonoid derivative having the hydroxy groups with an alkylating reagent such as an alkyl halide compound in the presence of a base. The alkyl group in the alkyl halide compound includes methyl groups, ethyl groups, n-propyl groups, n-butyl groups, n-octyl groups, benzyl groups and allyl groups. The same method as the above coupling method may also be applied to the coupling reaction of the alkyl-etherified biflavonoid derivative with the sialic acid derivative.

Moreover, each hydroxyl group moiety of the biflavonoid derivative (V) that is used as a raw material for coupling, for example, may also be converted to an alkyl group using standard methods. The alkyl group includes, for example, methyl groups.

Specifically, for example, a method using diazomethane may be used as an alkylation method. Thus, a biflavonoid derivative, in which a hydroxy group moiety mentioned above has been converted to an alkyl group, may be obtained. For example, at least one of R¹, R², R³, R⁴, R⁶, R⁷, and R⁸ may be converted to an alkyl group such as a methyl group. The same method as the above coupling method may also be applied to the coupling reaction of the alkylated biflavonoid derivative with the sialic acid derivative.

As is clear from the Examples, since the biflavonoid-sialic acid conjugates, in particular the compounds of the general formulae (I) to (III) or salts thereof of the present invention, have inhibitory activity against the influenza virus sialidase and anti-influenza virus activity, they may be used as pharmaceutical agents, like anti-influenza virus agents that use these activities. Moreover, the compounds may also be used for food and drink products, foods and beverages for specified health uses, healthy drinks, healthy foods, nutritional foods, and other various kinds of food and drink products.

When used for pharmaceutical agents, the compounds of the present invention may be administered into humans or animals, directly or with pharmaceutically acceptable conventional carriers, and either orally or parenterally. The compounds of the present invention may also be applied to various dosage forms, for example, orally administered forms including powders, granules, tablets, sugar-coated tablets, capsules, and ampules; injectables for subcutaneous, intramuscular, or intravenous injections; nasal drops; inhalations; and suppositories. These formulations may be produced by formulating the compounds alone or in combination with appropriate excipients, fillers, binders, moistening agents, disintegrants, detergents, lubricants, dispersing agents, buffers, preservatives, corrigents, flavoring agents, and coating agents. The anti-influenza virus agents thus obtained may generally be administered to an adult at a dose of 0.6 to 300 mg of compound per day, and preferably 5 to 200 mg/day, which is preferably divided into three to four doses per day, although the dose may differ depending on age, body weight, or symptoms of the patient, or administration route.

When used as food and drink products, the compounds, which are active ingredients, may be used alone or in combination with other foods or food components, according to standard methods. While the food and drink products using the active ingredients of the present invention may be in any form, including solids (powders, granules, or others), pastes, liquids, and suspensions, healthy drinks may also be produced using sweeteners, acidifiers, vitamins, and other components used for producing drink preparations.

### [Examples]

Herein below, the present invention will be specifically described using examples, however, it is not to be construed as limited thereto. As used herein, the term "room temperature" refers to a temperature preferably ranging from 20 to 30°C, and more preferably around 25°C. In addition, the term "around the clock" refers to about 24 hours.

### [Example 1] Separation and purification of biflavonoid components

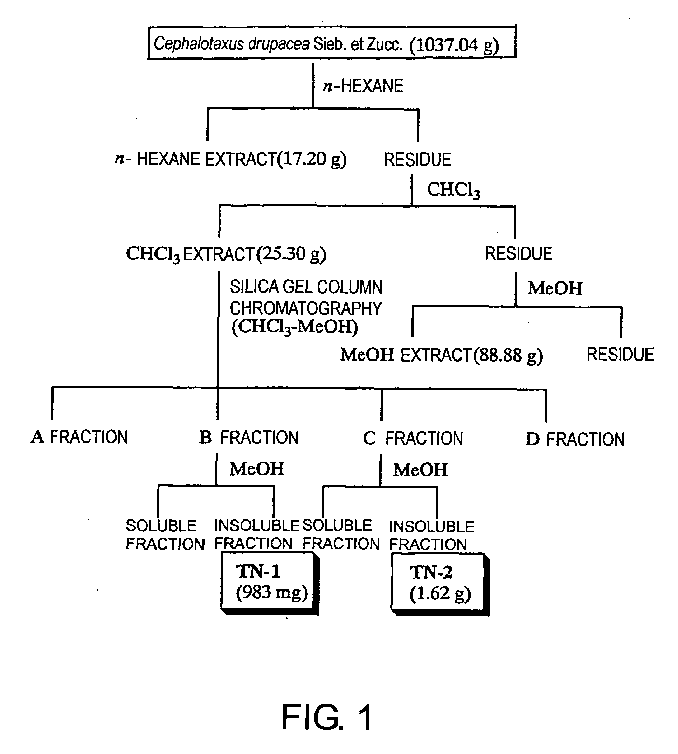

1037.04 g of *Cephalotaxus drupacea* Sieb. et Zucc. (Cephalotaxaceae) leaves, which had been left in a room until dry, was twice extracted using *n*-hexane, CHCl₃, and MeOH, in that order, each at room temperature and around the clock; the CHCl₃ extract was separated according to the procedure in Fig. 1. As a result, 983 mg and 1.62 g of two compounds, tentatively named TN-1 and TN-2, were respectively isolated and then identified as amentoflavone 7,4',7"-tri-*O*-methyl ether (**19**) and ginkgetin (**20**), respectively. Ginkgetin (**20**) can be prepared from *Ginkgo biloba* L. (Ginkgoaceae), *Cephalotaxus koreana, Cephalotaxus griffithii, Cephalotaxus harringtonia* C.koch (Cephalotaxaceae, heretofore), *Taxus caspidata* Sieb.et Zucc., *Taxus caspidata* Sieb.et Zucc. var. nana Rehder, *Taxus baccata, Torreya nucifera* Sieb. et Zucc. (Taxaceae, heretofore), *Lonicera japonica* (Caprifoliaceae), *Podocarpus macrophylla* (Podocarpaceae), *Selaginella moellendorfii* (Selaginellaceae), and additionally from *Callitris canescens* and *Zamia angustifolia.*

Melting point (mp) was determined using the Yanagimoto micro melting point apparatus and was uncorrected. Ultraviolet (UV) absorption spectra, infrared (IR) absorption spectra, and mass spectra (MS) were measured respectively using a Shimazu UV-240 instrument, JASCO A-102 instrument, and JEOL JMS-DX302 instrument. Nuclear magnetic resonance (NMR) spectra was measured using JEOL GSX-400 and JNM-LA500 instruments with TMS (tetramethyl silane) as a internal standard. Silica gel column chromatography was carried out using the Merck Kieselgel 60 (70-230 mesh).

### TN-1: Amentoflavone 7,4',7"-tri-O-methyl ether (19)

Pale yellow powder (983 mg); mp > 300°C, UV λₘₐₓ (EtOH) (logs): 207 (4.61), 270 (4.51), 330 (4.51); IR νₘₐₓ (KBr) cm⁻¹: 3200, 2950, 1660, 1605, 1500, 1440, 1205; EIMS *m*/*z* (rel. int. %): 580 (M⁺, 100), 549 (9), 503 (2), 355 (3), 221 (3); HREIMS *m*/*z*: 580.1368, calcd for C₃₃H₂₄O₁₀, 580.1369; ¹H-NMR (DMSO-*d*₆) δ: 7.02 (1H, s, 3-C), 6.38 (1H, d, J = 2.0 Hz, 6-C); 6.80 (1H, d, J = 2.0 Hz, 8-C), 8.12 (1H, d, J = 2.4 Hz, 2'-H), 7.38 (1H, d, J = 8.7 Hz, 5'-H), 8.24 (1H, dd, J = 2.4, 8.7 Hz, 6'-H), 6.86 (1H, s, 3"-H), 6.68 (1 H, s, 6"-H), 7.51 (2H, d, J = 9.1 Hz, 2"'-H, 6"'-H), 6.72 (2H, d, J = 9.1 Hz, 3"'-H, 5"'-H), 12.91 (1H, s, 5-OH), 13.26 (1H, s, 5"-OH), 10.31 (1H, bs, 4"'-OH), 3.83 (3H, s, 7-OCH₃), 3.79 (3H, s, 4'-OCH₃), 3.84 (3H, s, 7"-OCH₃), and ¹³C-NMR (DMSO-*d*_{*6*}) δ: 163.5 (2-C), 103.9 (3-C), 181.9 (4-C), 161.1 (5-C), 98.1 (6-C), 165.2 (7-C), 92.7 (8-C), 157.3 (9-C), 104.7 (10-C), 122.4 (1'-C), 130.8 (2'-C), 121.3 (3'-C), 160.5 (4'-C), 111.8 (5'-C), 128.4 (6'-C), 163.9 (2"-C), 102.5 (3"-C), 182.2 (4"-C), 161.5 (5"-C), 95.5 (6"-C), 165.5 (7"-C), 104.5 (8"-C), 153.4 (9"-C), 104.0 (10"-C), 121.1 (1"'-C), 128.1 (2"'-C, 5"'-C), 115.8 (3"'-C, 5"'-C), 161.2 (4"'-C), 56.0 (7-OCH₃), 56.0 (4'-OCH₃), 56.5 (7"-OCH₃).

### TN-2: Ginkgetin (20)

Pale yellow powder (1.62 g); mp 221-225°C; UV λₘₐₓ (EtOH) (logε): 207 (4.64), 270 (4.54), 330 (4.51); IR νₘₐₓ (KBr) cm⁻¹: 3300,2930,1650,1600,1490,1250; EIMS *m*/*z* (rel. int. %): 566 (M⁺, 100), 535 (8), 429 (2), 283 (5), 269 (11), 255 (2); HREIMS *m*/*z*: 566.1211, calcd for C₃₂H₂₂O₁₀, 566.1213; ¹H-NMR (DMSO-*d*₆) δ: 6.99 (1H, s, 3-H), 6.35 (1H, d, J = 2.0 Hz, 6-H), 6.78 (1H, d, J = 2.0 Hz, 8-H), 8.09 (1H, d, J = 2.4 Hz, 2'-H), 7.35 (1H, d, J = 8.7 Hz, 5'-H), 8.20 (1H, dd, J = 2.4, 8.7 Hz, 6'-H), 6.79 (1H, s, 3"-H), 6.40 (1H, s, 6"-H), 7.48 (2H, d, J = 9.1 Hz, 2"'-H, 6"'-H), 6.71 (2H, d, J = 9.1 Hz, 3"'-H, 5"'-H), 12.91 (1H, s, 5-OH), 13.22 (1H, s, 5"-OH), 10.83 (1H, bs, 7"-OH), 10.28 (1H, bs, 4"'-OH), 3.83 (3H, s, 7-OCH₃), 3.78 (3H, s, 4'-OCH₃), ¹³C-NMR (DMSO-*d*_{*6*}) δ: 163.2 (2-C), 103.6 (3-C), 181.9 (4-C), 160.6 (5-C), 98.1 (6-C), 165.2 (7-C), 92.7 (8-C), 157.3 (9-C), 104.7 (10-C), 122.3 (1'-C), 130.9 (2'-C), 121.6 (3'-C), 160.6 (4'-C), 111.7 (5'-C), 128.4 (6'-C), 163.5 (2"-C), 102.5 (3"-C), 182.2 (4"-C), 161.1 (5"-C), 98.6 (6"-C), 161.7 (7"-C), 103.6 (8"-C), 154.3 (9"-C), 103.8 (10"-C), 121.2 (1"'-C), 128.0 (2"'-C, 5"'-C), 115.8 (3"'-C, 5"'-C), 161.1 (4"'-C), 56.0 (7-OCH₃), 55.9 (4'-OCH₃).

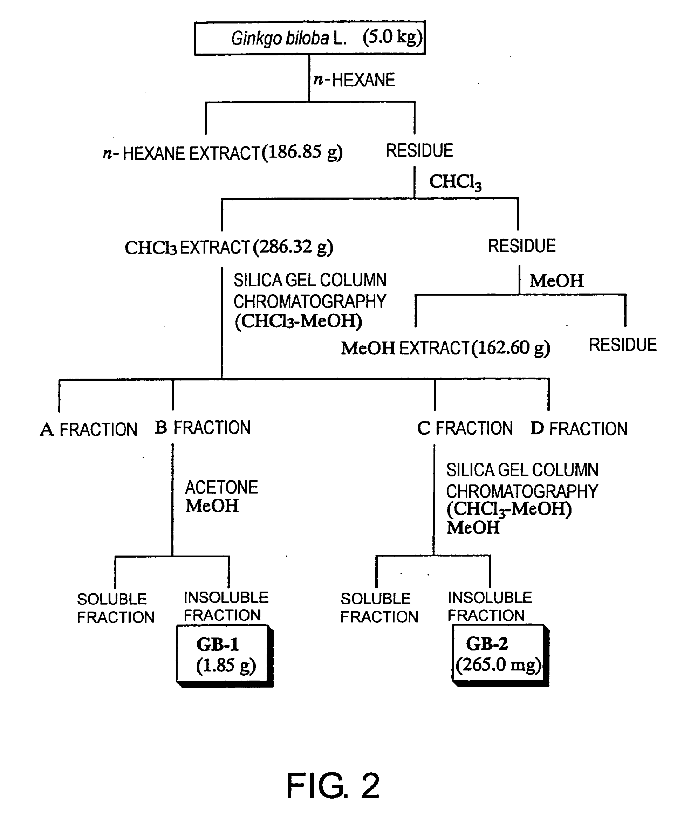

5.0 kg of *Ginkgo biloba* L. leaves, which had been left in a room until dry, was twice extracted using n-hexane, CHCl₃, and MeOH, in that order, each at room temperature and around the clock; the CHCl₃ extract was separated according to the procedure in Fig. 2. As a result, 1.85 g and 265 mg of two compounds, tentatively named GB-1 and GB-2, were respectively isolated, and then identified as sciadopitysin (**21**) and isoginkgetin (**22**), respectively.

### GB-1: Sciadopitysin (21)

Pale yellow powder (1.85 g); mp 295-298°C; UV λₘₐₓ (EtOH) (logε): 205 (4.41), 270 (4.30), 325 (4.28); IR νₘₐₓ (KBr) cm⁻¹ (rel. int. %): 3350, 1700, 1650, 1550, 1480, 1420, 1210; EIMS *m*/*z:* 580 (M⁺, 100), 551 (9),290 (11), 167 (4), 135 (6); HREIMS *m*/*z:* 580.1373, calcd for C₃₃H₂₄O₁₀, 580.1369; ¹H-NMR (DMSO-*d*_{*6*}) δ: 6.99 (1H, s, 3-H), 6.36 (1H, d, J = 2.0 Hz, 6-H), 6.78 (1H, d, J = 2.0 Hz, 8-H), 8.09 (1H, d, J = 2.4 Hz, 2'-H), 7.36 (1H, d, J = 8.7 Hz, 5'-H), 8.23 (1H, dd, J = 2.4, 8.7 Hz, 6'-H), 6.90 (1H, s, 3"-H), 6.43 (1H, s, 6"-H), 7.60 (2H, d, J = 8.8 Hz, 2"'-H, 6"'-H), 6.93 (2H, d, J = 8.8 Hz, 3"'-H, 5"'-H), 12.97 (1H, s, 5-OH), 13.07 (1H, s, 5"-OH), 10.88 (1H, bs, 7"-OH), 3.82 (3H, s, 7-OCH₃), 3.81 (3H, s, 4'-OCH₃), 3.76 (3H, s, 4"'-OCH₃), ¹³C-NMR (DMSO-*d*_{*6*}) δ: 163.5 (2-C), 103.7 (3-C), 181.9 (4-C), 161.0 (5-C), 98.0 (6-C), 165.1 (7-C), 92.6 (8-C), 157.2 (9-C), 104.7 (10-C), 122.3 (1'-C), 130.8 (2'-C), 121.5 (3'-C), 160.5 (4'-C), 111.6 (5'-C), 128.2 (6'-C), 163.0 (2"-C), 103.1 (3"-C), 182.0 (4"-C), 160.6 (5"-C), 98.5 (6"-C), 161.7 (7"-C), 103.5 (8"-C), 154.2 (9"-C), 103.5 (10"-C), 122.7 (1"'-C), 127.7 (2"'-C, 6"'-C), 114.4 (3"'-C, 5"'-C), 162.1 (4"'-C), 55.8 (7-OCH₃), 55.9 (4'-OCH₃), 55.4 (4"'-OCH₃).

### GB-2: Isoginkgetin (22)

Pale yellow powder (265 mg); mp 219-222°C; UV λₘₐₓ (EtOH): 205 (5.00), 270 (4.89), 330 (4.81); IR νₘₐₓ (KBr) cm⁻¹: 3400, 1650, 1600, 1500, 1240; EIMS *m*/*z* (rel. int. %): 566 (M⁺, 100), 551 (5), 537 (10), 283 (10), 153 (3), 135 (8); HREIMS *m*/*z*: 566.1211, calcd for C₃₂H₂₂O₁₀, 566.1213; ¹H-NMR (DMSO-*d*_{*6*}) δ: 6.92 (1H, s, 3-H), 6.21 (1H, d, J = 2.0 Hz, 6-H), 6.49 (1H, d, J = 2.0 Hz, 8-H), 8.07 (1H, d, J = 2.4 Hz, 2'-H), 7.36 (1H, d, J = 9.1 Hz, 5'-H), 8.19 (1H, dd, J = 2.4, 9.1 Hz, 6'-H), 6.90 (1H, s, 3"-H), 6.43 (1H, s, 6"-H), 7.61 (2H, d, J = 9.1 Hz, 2"'-H, 6"'-H), 6.94 (2H, d, J = 9.1 Hz, 3"'-H, 5"'-H), 12.94* (1H, s, 5-OH), 13.08* (1H, s, 5"-OH), 10.84 (1H, bs, 7-OH), 10.84 (1H, bs, 7"-OH), 3.80 (3H, s, 4'-OCH₃), 3.76 (3H, s, 4"'-OCH₃), ¹³C-NMR (DMSO-*d*_{*6*}) δ: 163.2 (2-C), 103.6 (3-C), 181.7 (4-C), 161.7 (5-C), 98.8 (6-C), 163.0 (7-C), 94.0 (8-C), 157.3 (9-C), 103.7* (10-C), 122.5 (1'-C), 130.8 (2'-C), 121.5 (3'-C), 160.5 (4'-C), 111.6 (5'-C), 128.1 (6'-C), 164.1 (2"-C), 103.1 (3"-C), 182.0 (4"-C), 160.3 (5"-C), 98.5 (6"-C), 161.4 (7"-C), 103.6* (8"-C), 154.3 (9"-C), 103.6 (10"-C), 122.7 (1"'-C), 127.7 (2"'-C, 6"'-C), 114.4 (3"'-C, 5"'-C), 162.1 (4"'-C), 55.8 (4'-OCH₃), 55.4 (4"'-OCH₃). *: may be interchanged.

### [Example 2] Synthesis of sialyl biflavonoids

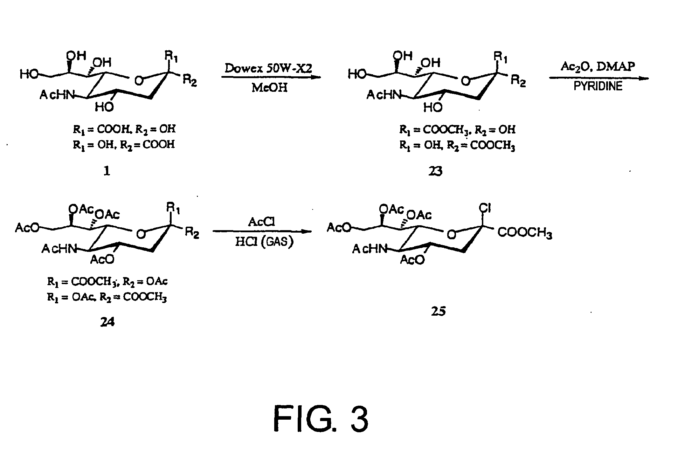

### 1. Synthesis of sugar donor (2β-chloride) (25) (Fig. 3)

200 ml of MeOH (dried) was added to sialic acid (*N*-acetylneuraminic acid) (1) (5.0 g, 16 mmol, provided by Kitasato University, School of Pharmaceutical Sciences) and stirred for one hour. To this solution, a molecular sieve (30.0 g) and Dowex 50W-X2 (Dow Chemical Co., 100-200 mesh) were added and the mixture was stirred for 24 hours at room temperature in air. After the reaction was completed, the molecular sieve and Dowex 50W-X2 were removed by centrifugation and filtration, followed by concentrating in vacuo to remove the solvent, yielding 1-methyl-*N*-acetylneuraminic acid (23) (4.80 g, 92%). (The term "compound (23) " may be simply expressed as "23" hereinafter. The same is true for other compounds hereinafter.)

23 (4.80 g) thus obtained was dissolved in 150 ml pyridine (dried), and 120 ml of acetic anhydride (1.27 mol), and 4-dimethylaminopyridine (DMAP) (18-36 mg, 0.149-0.298 mmol) as a catalyst was added to the solution, followed by stirring for 24 hours at room temperature in air. After the reaction, the reacted solution was concentrated in vacuo in the presence of toluene to completely remove any smell from the pyridine and acetic acid. After separation and purification of the reaction product by column chromatography (a CHCl₃-isopropanol solvent system), 1-methyl-2,4,7,8,9-penta-*O*-acetyl-*N*-acetylneuraminic acid **(24)** (6.59 g, 83.2%) was obtained. **24** (6.59 g) was dissolved in 150 ml of acetyl chloride, and the solution was bubbled with HCl gas for ten minutes under ice-cold conditions, and then stirred at room temperature for 24 hours. After the reaction, the solution was azeotroped with CHCl₃, yielding 2-chloro-1-methyl-4,7,8,9-tetra-*O*-acetyl-*N*-acetylneuraminic acid) **(25)** (6.20 g, 98.4%).

### 1-Methyl-N-actylneuraminic acid (23);

IR νₘₐₓ (KBr) cm⁻¹: 3350, 2940, 1740, 1640, 1550, 1430, 1370, 1010; positive FABMS *m*/*z* (rel. int. %): 324 ([M+H]⁺, 11), 309 (16); ¹H-NMR (DMSO-*d*_{*6*}) δ: 1.72 (1H, d, J = 12.1 Hz), 1.89 (3H, s), 2.03 (1H, dd, J = 12.6, 4.7 Hz), 3.70 (3H, s), 3.3-6.5 (13H, m), 8.18 (1H, d, J = 8.0 Hz), ¹³C-NMR (DMSO-*d*_{*6*}) δ: 22.4, 48.5, 52.2, 53.0, 63.5, 65.3, 69.0, 69.5, 70.4, 94.8, 170.1, 171.9.

### 1-Methyl-2,4,7,8,9-penta-O-acetyl-N-acetylneuraminic acid (24);

IR νₘₐₓ (KBr) cm⁻¹: 3300, 2900, 1750, 1670, 1550, 1450, 1380, 1230, 1050; positive FABMS *m*/*z* (rel. int. %): 534 ([M+H]⁺, 3), 474 (9), 414 (34); ¹H-NMR (DMSO-*d*_{*6*}) δ: 1.70 (s, α-NHCOCH₃), 1.70 (s, β-NHCOCH₃), 1.94-2.10 (s, α-OCOCH₃), 1.94-2.13 (s, β-OCOCH₃), 1.90 (m), 2.42 (dd, J = 5.0, 13.5 Hz, β-H-3eq), 2.66 (dd, J = 5.0, 12.9 Hz, α-H-3eq), 3.68 (s, β-COOCH₃), 3.70 (s, α-COOCH₃), 3.97 (m), 4.11 (d, J = 10.5 Hz), 4.26 (m), 4.95 (m), 5.05 (m), 5.20 (m), 7.81 (d, J = 9.3 Hz, β-NH), 7.87 (d, J = 9.3 Hz, α-NH), ¹³C-NMR (DMSO-*d*_{*6*}) δ: α 20.4-20.6, 22.5, 35.9, 47.5, 52.7, 61.5, 66.8, 68.6, 68.6, 72.5, 96.3, 167.5, 168.3, 169.1, 169.6, 169.9, β 20.4-20.6, 22.5, 35.6, 47.4, 52.8, 61.3, 66.8, 68.5, 69.1, 71.5, 96.7, 166.0, 168.0, 168.9, 169.0, 169.2, 169.7, 169.9.

### 2-Chloro-1-methyl-4,7,8,9-tetra-O-acetyl-N-acetylneuraminic acid (25);

IR νₘₐₓ (KBr) cm⁻¹ : 3300, 2860, 1750, 1660, 1540, 1440, 1370, 1220, 1035; positive FABMS *m*/*z* (rel. int. %): 510 ([N4+H]⁺, 11.7), 492 (21), 474 (8), 450 (7), 432 (12), 414 (55), 372 (9), 252 (7), 196 (7); ¹H-NMR (DMSO-*d*_{*6*}) δ: 1.72 (3H, s), 1.96 (3H, s), 1.99 (3H, s), 2.01 (6H, s), 2.32 (1H, dd, J = 11.1, 13.9 Hz), 2.69 (1H, dd, J = 4.4, 13.9 Hz), 3.79 (3H, s), 4.01 (2H, m), 4.24 (1H, bd, J = 12.3 Hz), 4.39 (1H, bd, J = 10.7 Hz), 5.06 (m), 5.17 (m), 5.32 (1H, d, J = 11.9 Hz), 7.95 (1H, d, J = 9.5 Hz).

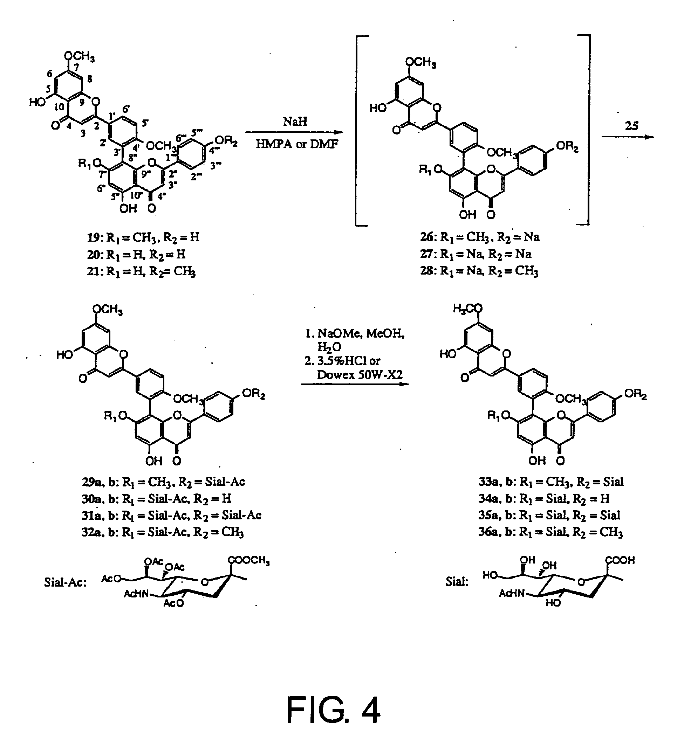

### 2. Synthesis of acetyl-protected sialyl biflavonoids (Fig. 4)

Each of amentoflavone 7,4',7"-tri-*O*-methyl ether (**19**) (500 mg, 0.86 mmol), ginkgetin (**20**) (1.0 g, 1.77 mmol), and sciadopitysin (**21**) (1.0 g, 1.72 mmol) was dissolved in 30 ml of dimethyl formamide (DMF), and sodium hydride (NaH) (36.0 mg, 1.50 mmol, 90.0 mg, 3.75 mmol, and 40 mg, 1.67 mmol, respectively) was added to the solution. After reacting for two hours, **25** (600 mg, 1.18 mmol, 2.0 g, 3.92 mmol, and 1.0 g, 1.96 mmol, respectively) was further added to each solution, and this was reacted for 24 hours at room temperature in air. After the reaction was complete, CHCl₃ was added to the reaction solution and washed with saturated NaCl solution, and then with saturated NaHCO₃ solution, dehydrated with anhydrous Na₂SO₄, and then concentrated in vacuo. The concentrate was separated using a column of Sephadex LH-20 (Amersham Biosciences) and HPLC (SENSHU PAK PEGASIL Silica 60-5, 10φx 250 mm), to yield **29a** (198.7 mg, 21.9%) and **29b** (248.5 mg, 27.4%) from **19, 30a** (97.9 mg, 5.3%), **30b** (73.4 mg, 4.0%), **31a** (216.5 mg, 8.1%), and **31b** (323.5 mg, 12.1%) from **20,** and **32a** (234.9 mg, 12.9%) and **32b** (367.9 mg, 20.2%) from **21.**

When a non-empirical determination method using ¹³C-NMR with LSPD (long range selective proton decoupling) (H. Hori, T. Nakajima, Y Nishida, H. Ohrui, H. Meguro, Tetrahedron Lett., 29: 6317-6320 (1988)) was applied and examined, all biflavonoids acetylated sialic acid conjugates were found to be α-anomers in their stereochemistry.

CD spectral analysis of these compounds revealed that the biflavonoid moieties of **29a** and **29b, 30a** and **30b, 31a** and **31b,** and **32a** and **32b** are each atropisomers. In addition, CD (circular dichroism) was determined using the following instrument under the conditions below:
- Instrument:: JASCO J-720 Circular Dichroism Spectropolarimeter
- Conditions:: Cell length 0.5 cm
Concentration 0.02%
Solvent EtOH
Temperature 20°C
Range 450 nm-200 nm
Band width 1.0 nm
Resolution 1 nm
Accumulation 1
Sensitivity 200 mdeg
Response 1 sec
Speed 50 nm/min

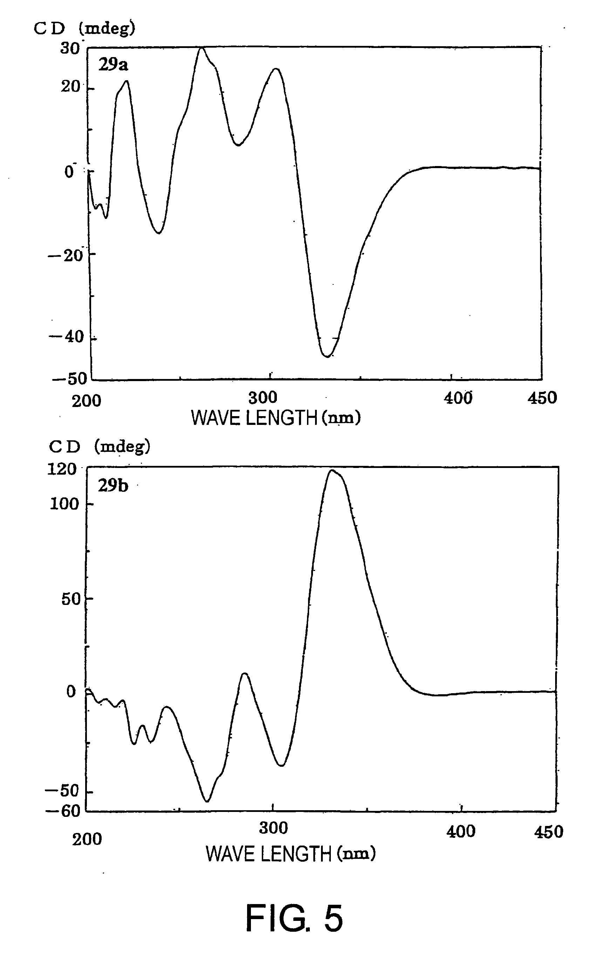

The CD spectrum of **29a** shows a positive first Cotton effect at 331 nm and a negative second Cotton effect at 305 nm due to the *p*-methoxycinnamoyl moiety, indicating a positive exciton chirality, and revealing that the two transition moments are configured clockwise (Fig. 5). Thus, the stereochemistry of the inter-flavone bond in the biflavonoid moiety was determined to be the *R* configuration. In addition, because the CD spectrum around 265 nm due to the *p*-methoxybenzoyl chromophore indicated a positive first Cotton at 285 nm and a negative second Cotton at 270 nm, the stereochemistry was also determined to be *R* configuration.

On the other hand, the CD spectrum of **29b** shows a negative first Cotton effect at 332 nm and a positive second Cotton effect at 305 nm due to the *p*-methoxycinnamoyl moiety, indicating a negative exciton chirality, and the two transition moments are configured counter clockwise (Fig. 5). Thus, the stereochemistry of the inter-flavone bond in the biflavonoid moiety was determined to be the S configuration. In addition, because the CD spectrum around 270 nm due to the *p*-methoxybenzoyl chromophore indicated a negative first Cotton at 284 nm and a positive second Cotton at 270 nm, the stereochemistry was also determined to be S configuration.

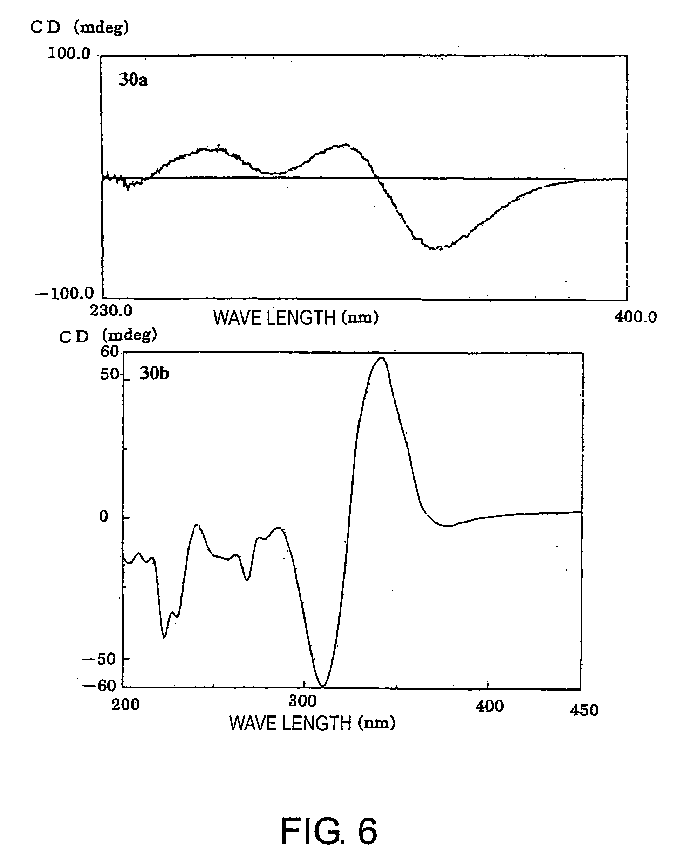

The CD spectrum of **30a** shows a negative first Cotton effect at 338 nm and a positive second Cotton effect at 309 nm due to the *p*-methoxycinnamoyl moiety, indicating a negative exciton chirality, and revealing that the two transition moments are configured counter clockwise (Fig. 6). Thus, stereochemistry of the inter-flavone bond in the biflavonoid moiety was determined to be the *R* configuration.

On the other hand, the CD spectrum of **30b** shows a positive first Cotton effect at 341 nm and a negative second Cotton effect at 310 nm due to the *p*-methoxycinnamoyl moiety, indicating a positive exciton chirality, and revealing that the two transition moments are configured clockwise (Fig.6). Thus, stereochemistry of the inter-flavone bond in the biflavonoid moiety was determined to be the *S* configuration. In addition, because the CD spectrum around 270 nm due to the *p*-methoxybenzoyl chromphore indicated a positive first Cotton at 286 nm and a negative second Cotton at 272 nm, the stereochemistry was also determined to be S configuration.

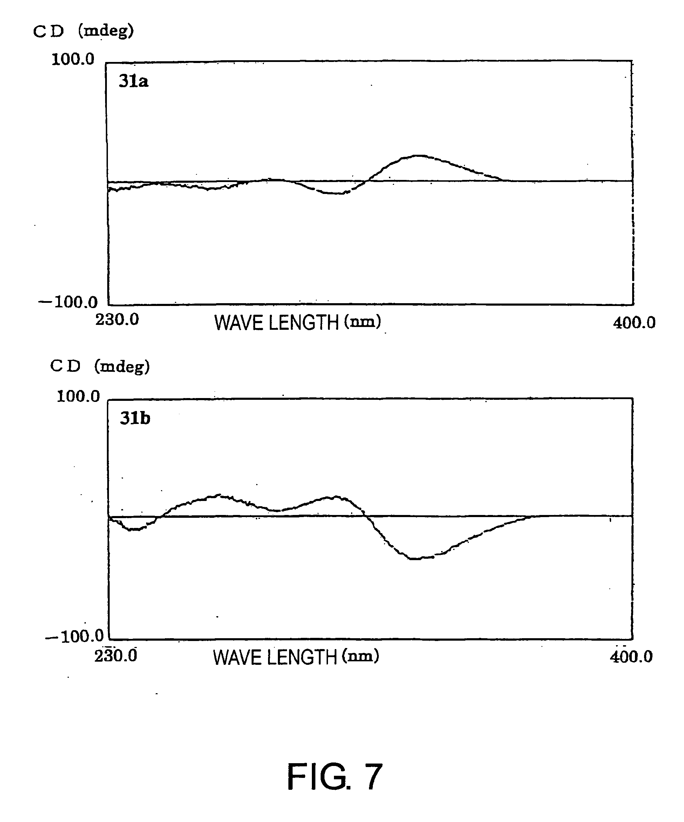

The CD spectrum of **31a** shows a positive first Cotton effect at 332 nm and a negative second Cotton effect at 305 nm due to the *p*-methoxycinnamoyl moiety, indicating a positive exciton chirality, and revealing that the two transition moments are configured clockwise (Fig. 7). Thus, stereochemistry of the inter-flavone bond in the biflavonoid moiety was determined to be the S configuration.

On the other hand, the CD spectrum of **31b** shows a negative first Cotton effect at 330 nm and a positive second Cotton effect at 304 nm due to the *p*-methoxycinnamoyl moiety, indicating a negative exciton chirality, and revealing that the two transition moments are configured counter clockwise (Fig. 7). Thus, stereochemistry of the inter-flavone bond in the biflavonoid moiety was determined to be the *R* configuration.

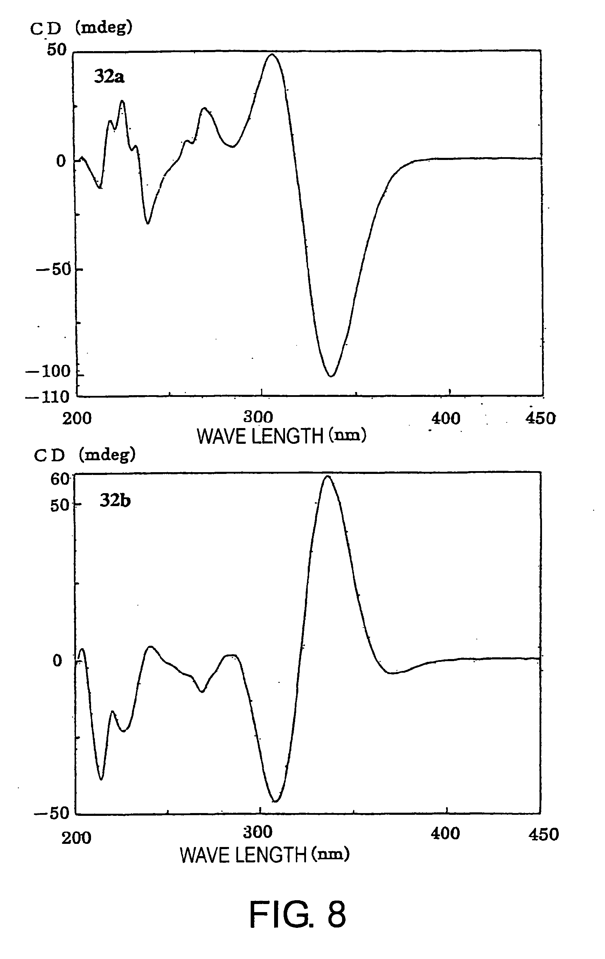

The CD spectrum of **32a** gave a negative first Cotton effect at 337 nm and a positive second Cotton effect at 307 nm due to the *p*-methoxycinnamoyl moiety, indicating a negative exciton chirality, and revealing that the two transition moments are configured counter clockwise (Fig. 8). Thus, stereochemistry of the inter-flavone bond in the biflavonoid moiety was determined to be the *R* configuration. In addition, because the CD spectrum around 270 nm due to the *p*-methoxybenzoyl chromophore indicated a negative first Cotton at 286 nm and a positive second Cotton at 270 nm, the stereochemistry was also determined to be *R* configuration.

On the other hand, the CD spectrum of **32b** shows a positive first Cotton effect at 336 nm and a negative second Cotton effect at 308 nm due to the *p*-methoxycinnamoyl moiety, indicating a positive exciton chirality, and revealing that the two transition moments are configured clockwise (Fig. 8). Thus, stereochemistry of the inter-flavone bond in the biflavonoid moiety was determined to be the S configuration. In addition, because the CD spectrum around 270 nm due to the *p*-methoxybenzoyl chromophore indicated a positive first Cotton at 280 nm and a negative second Cotton at 270 nm, the stereochemistry was also determined to be S configuration.

These results revealed that the biflavonoid moieties of **29a** and **29b, 30a** and **30b, 31a** and **31b,** and **32a** and **32b** are each atropisomers.

### (R)-7,4',7"-tri-O-methyl-4"'-O-(1""-methyl-4"",7"",8"",9""-tetra-O-acetyl-N-acetylneuramin yl)amentoflavone (29a)

Pale yellow powder; mp 162-167°C; UV λₘₐₓ (EtOH) (logε): 210 (4.73), 270 (4.66), 320 (4.53); IR νₘₐₓ (KBr) cm⁻¹: 3450, 3050, 1760, 1680, 1620, 1510, 1440, 1380, 1230, 1040, 850; positive FABMS *m*/*z* (rel. int. %): 1054 ([M+H]⁺, 14), 565 (10), 535 (4), 474 (6), 372 (4), 252 (8); ¹H-NMR (DMSO-*d*_{*6*}) δ: biflavonoid moiety; 6.98 (1H, s, 3-H), 6.33 (1H, d, J = 2.1 Hz, 6-H), 6.73 (1H, d, J = 2.1 Hz, 8-H), 8.09 (1H, d, J = 2.4 Hz, 2'-H), 7.37 (1H, d, J = 8.9 Hz, 5'-H), 8.21 (1H, dd, J = 8.9, 2.4 Hz, 6'-H), 6.97 (1H, s, 3"-H), 6.67 (1H, s, 6"-H), 7.61 (2H, d, J = 8.9 Hz, 2"'-H, 6"'-H), 7.04 (2H, d, J = 8.9 Hz, 3"'-H, 5"'-H), 12.90 (1H, s, 5-OH), 13.16 (1H, s, 5"-OH), 3.79 (3H, s, 7-OCH₃), 3.82 (3H, s, 4'-OCH₃), 3.71 (3H, s, 7"-OCH₃), sialic acid moiety; 2.07 (1H, m, 3""-H), 2.64 (1H, dd, J = 12.8,4.6 Hz, 3""-H), 4.77 (1H, dt, J = 11.0, 4.6 Hz, 4""-H), 3.94 (1H, m, 5""-H), 4.43 (1H, d, J = 11.0 Hz, 6""-H), 5.19 (1H, m, 7""-H), 5.19 (1H, m, 8""-H), 4.03 (1H, dd, J =11.0,4.0 Hz, 9""-H), 4.15 (1H, dd, J = 11.0, 2.4 Hz, 9""-H), 7.80 (1H, d, J = 9.8 Hz, NH), 3.55 (3H, s, COOCH₃), 1.93 (3H, OCOCH₃), 1.98 (3H, OCOCH₃), 1.99 (3H, OCOCH₃), 2.06 (3H, OCOCH₃), 1.72 (3H, s, NHCOCH₃), ¹³C-NMR (DMSO-*d*_{*6*}) δ: biflavonoid moiety; 163.5 (2-C), 104.2^{*2} (3-C), 181.9 (4-C), 161.1 (5-C), 97.9 (6-C), 165.1 (7-C), 92.6 (8-C), 157.2 (9-C), 104.7*¹ (10-C), 122.4 (1'-C), 130.6 (2'-C), 121.0 (3'-C), 160.3 (4'-C), 112.0 (5'-C), 128.3 (6'-C), 162.9 (2"-C), 104.1*² (3"-C), 182.2 (4"-C), 161.5 (5"-C), 95.6 (6"-C), 162.6 (7"-C), 104.7*¹ (8"-C), 153.5 (9"-C), 103.7 (10"-C), 125.5 (1"'-C), 127.7 (2"'-C, 6"'-C), 118.8 (3"'-C, 5"'-C), 156.4 (4"'-C), 56.4 (7-OCH₃), 55.9*³ (4'-OCH₃), 55.8*³ (7"-OCH₃), sialic acid; 167.7 (1""-C), 99.3 (2""-C), 38.0 (3""-C), 68.2 (4""-C), 47.6 (5""-C), 72.8 (6""-C), 66.7 (7""-C), 67.5 (8""-C), 61.6 (9""-C), 53.0 (COOCH₃), 20.45 (2C, OCOCH₃), 20.47 (OCOCH₃), 20.6 (OCOCH₃), 22.5 (NHCOCH₃), 169.1 (OCOCH₃), 169.2 (OCOCH₃), 169.6 (OCOCH₃), 170.9 (OCOCH₃), 169.1 (NHCOCH₃). *¹, *², *³: may be interchanged.

### (S)-7,4',7"-tri-O-methyl-4"'-O-(1""-methyl-4'''', 7'''', 8'''', 9''''-tetra-O-acetyl-N-acetylneuramin yl)amentoflavone (29b)

Pale yellow powder; mp 161-168°C; UV λₘₐₓ (EtOH) (logs): 213 (4.78), 270 (4.73), 320 (4.61); IR νₘₐₓ (KBr) cm⁻¹: 3300, 3000, 1760, 1660, 1610, 1510, 1440, 1380, 1350, 1210, 1040, 840; positive FABMS *m*/*z* (rel. int. %): 1054 ([M+H]⁺, 13), 565 (5), 414 (12), 252 (4); ¹H-NMR (DMSO-*d*_{*6*}) δ: biflavonoid moiety; 6.98 (1H, s, 3-H), 6.33 (1H, d, J = 2.1 Hz, 6-H), 6.73 (1H, d, J = 2.1 Hz, 8-H), 8.09 (1H, d, J = 2.4 Hz, 2'-H), 7.37 (1H, d, J = 8.9 Hz, 5'-H), 8.21 (1H, dd, J = 8.9,2.4 Hz, 6'-H), 6.97 (1H, s, 3"-H), 6.67 (1H, s, 6"-H), 7.61 (2H, d, J = 8.9 Hz, 2"'-H, 6"'-H), 7.04 (2H, d, J = 8.9 Hz, 3"'-H, 5"'-H), 12.90 (1H, s, 5-OH), 13.16 (1H, s, 5"-OH), 3.79 (3H, s, 7-OCH₃), 3.82 (3H, s, 4'-OCH₃), 3.71 (3H, s, 7"-OCH₃), sialic acid moiety; 2.01 (1H, m, 3""-H), 2.66 (1H, dd, J = 12.8,4.9 Hz, 3""-H), 4.77 (1H, dt, J = 11.0, 4.9 Hz, 4""-H), 3.92 (1H, m, 5""-H), 4.40 (1H, d, J = 10.7 Hz, 6""-H), 5.17 (1H, d, J = 10.7 Hz, 7""-H), 5.13 (1H, m, 8""-H), 3.99 (1H, m, 9""-H), 3.99 (1H, m, 9""-H), 7.78 (1H, d, J = 9.8 Hz, NH), 3.60 (3H, s, COOCH₃), 1.93 (3H, OCOCH₃), 1.93 (3H, OCOCH₃), 1.95 (3H, OCOCH₃), 2.08 (3H, OCOCH₃), 1.70 (3H, s, NHCOCH₃), ¹³C-NMR (DMSO-*d*_{*6*}) δ: biflavonoid moiety; 163.5 (2-C), 103.7 (3-C), 181.8 (4-C), 161.1 (5-C), 97.9 (6-C), 165.1 (7-C), 92.5 (8-C), 157.2 (9-C), 104.7*¹ (10-C), 122.5 (1'-C), 130.6 (2'-C), 121.1 (3'-C), 160.3 (4'-C), 111.8 (5'-C), 128.3 (6'-C), 162.8 (2"-C), 104.2*¹ (3"-C), 182.2 (4"-C), 161.4 (5"-C), 95.6 (6"-C), 162.7 (7"-C), 104.6*¹ (8"-C), 153.5 (9"-C), 104.1*¹ (10"-C), 125.6 (1"'-C), 127.6 (2"'-C, 6"'-C), 119.1 (3"'-C, 5"'-C), 156.4 (4"'-C), 55.8*² (7-OCH³), 55.8*² (4'-OCH₃), 55.9*² (7"-OCH₃), sialic acid; 167.6 (1""-C), 99.4 (2""-C), 37.8 (3""-C), 68.3 (4""-C), 47.6 (5""-C), 72.8 (6""-C), 66.5 (7""-C), 67.6 (8""-C), 61.2 (9""-C), 53.1 (COOCH₃), 20.35 (OCOCH₃), 20.37 (OCOCH₃), 20.44 (OCOCH₃),20.6 (OCOCH₃),22.5 (NHCOCH₃),169.1 (OCOCH₃),169.2 (OCOCH₃),169.6 (OCOCH₃),169.8 (OCOCH₃),168.9 (NHCOCH₃). *¹, *²: may be interchanged.

### (R)-7,4'-di-O-methyl-7"-O-(1""-methyl-4"",7"".8"",9""-tetra-O-acetyl-N-acetylneuraminyl)a mentoflavone (30a)

Pale yellow powder; mp 160-166°C; UV λₘₐₓ (EtOH) (logε): 205 (4.61), 270 (4.47), 330 (4.46); IR νₘₐₓ (KBr) cm⁻¹: 3280,2880,1760,1660,1605,1510,1440,1370,1260,1220,1030, 840; positive FABMS *m*/*z* (rel. int. %): 1040 ([M+H)⁺, 6), 567 (23), 535 (4), 414 (5); ¹H-NMR (DMSO-*d*_{*6*}) δ: biflavonoid moiety; 7.00 (1H, s, 3-H), 6.38 (1H, d, J = 1.9 Hz, 6-H), 6.76 (1H, d, J = 1.9 Hz, 8-H), 8.16 (1H, d, J = 2.4 Hz, 2'-H), 7.38 (1H, d, J = 8.9 Hz, 5'-H), 8.26 (1H, dd, J = 8.9,2.4 Hz, 6'-H), 6.93 (1H, s, 3"-H), 6.96 (1H, s, 6"-H), 7.55 (2H, d, J = 8.9 Hz, 2"'-H, 6"'-H), 6.72 (2H, d, J = 8.9 Hz, 3"'-H, 5"'-H), 12.91 (1H, s, 5-OH), 13.15 (1H, s, 5"-OH), 10.33 (1H, bs, 4"'-OH), 3.84 (3H, s, 7-OCH₃), 3.80 (3H, s, 4'-OCH₃), sialic acid moiety; 1.94 (1H, m, 3""-H), 2.41 (1H, dd, J =13 .1, 4.9 Hz, 3""-H), 4.73 (1H, dt, J = 10.7,4.9 Hz, 4""-H), 3.92 (1H, m, 5""-H), 4.27 (1 H, m, 6""-H), 5.17 (1H, dd, J = 6.7, 1.5 Hz, 7""-H), 5.28 (1H, dt, J = 6.7, 3 .1 Hz, 8""-H), 4.11 (1H, dd, J =12.2, 6.7 Hz, 9""-H), 4.27 (1H, m, 9""-H), 7.76 (1H, t, J = 9.8 Hz, NH), 3.63 (3H, s, COOCH₃), 1.84 (3H, OCOCH₃), 1.99 (3H, OCOCH₃), 2.05 (3H, OCOCH₃), 2.07 (3H, OCOCH₃), 1.70 (3H, s, NHCOCH₃), ¹³C-NMR (DMSO-*d*₆) δ: biflavonoid moiety; 163.6 (2-C), 103.8 (3-C), 181.9 (4-C), 161.2 (5-C), 98.0 (6-C), 165.2 (7-C), 92.7 (8-C), 157.3 (9-C), 104.7 (10-C), 122.1 (1'-C), 130.8 (2'-C), 120.6 (3'-C), 160.4 (4'-C), 111.6 (5'-C), 128.6 (6'-C), 164.4 (2"-C), 102.4 (3"-C), 182.4 (4"-C), 160.4 (5"-C), 102.9 (6"-C), 156.2 (7"-C), 108.5 (8"-C), 153.6 (9"-C), 106.5 (10"-C), 120.9 (1"'-C), 128.2 (2"'-C, 6"'-C), 115.8 (3"'-C, 5"'-C), 161.3 (4"'-C), 56.0 (7-OCH₃), 55.8 (4'-OCH₃), sialic acid; 166.9 (1""-C), 100.0 (2""-C), 35.9 (3""-C), 68.4 (4""-C), 47.5 (5""-C), 73.0 (6""-C), 67.1 (7""-C), 68.5 (8""-C), 61.8 (9""-C), 53.1 (COOCH₃), 20.37 (2C, OCOCH₃), 20.44 (OCOCH₃), 20.5 (OCOCH₃), 22.5 (NHCOCH₃), 169.1 (OCOCH₃), 169.3 (OCOCH₃), 169.4 (OCOCH₃), 170.0 (OCOCH₃), 169.1 (NHCOCH₃).

### (S)-7,4'-di-O-methyl-7"-O-(1""-methyl-4"",7"",8"",9""-tetra-O-acetyl-N-acetyleuraminyl)a mentoflavone (30b)

Pale yellow powder; mp 176-179°C; UV λₘₐₓ (EtOH) (logε): 208 (4.71), 268 (4.61), 330 (4.61); IR νₘₐₓ (KBr) cm⁻¹: 3300, 2880, 1750, 1660, 1610, 1510, 1440, 1370, 1220, 1040, 840; positive FABMS *m*/*z* (rel. int. %): 1040 ([M+H]⁺, 7), 567 (30), 535 (6); ¹H-NMR (DMSO-*d*_{*6*}) δ: biflavonoid moiety; 6.97 (1H, s, 3-H), 6.36 (1H, d, J = 2.1 Hz, 6-H), 6.70 (1H, d, J = 2.1 Hz, H-8), 8.14 (1H, d, J = 2.4 Hz, 2'-H), 7.38 (1H, d, J = 8.9 Hz, 5'-H), 8.24 (1H, dd, J = 8.9,2.4 Hz, 6'-H), 6.93 (1H, s, 3"-H), 6.92 (1H, s, 6"-H), 7.54 (2H, d, J = 8.9 Hz, 2"'-H, 6"'-H), 6.73 (2H, d, J = 8.9 Hz, 3"'-H, 5"'-H), 12.88 (1H, s, 5-OH), 13.16 (1H, s, 5"-OH), 10.30 (1H, bs, 4"'-OH), 3.82 (3H, s, 7-OCH₃), 3.79 (3H, s, 4'-OCH₃), sialic acid moiety; 1.90 (1H, m, 3""-H), 2.39 (1H, dd, J = 13.1,4.9 Hz, 3""-H), 4.72 (1H, dt, J = 10.7, 4.9 Hz, 4""-H), 3.91 (1H, m, 5''''-H), 4.32 (1H, dd, J = 10.7, 1.8 Hz, 6""-H), 5.16 (1H, dd, J = 6.7, 1.8 Hz, 7""-H), 5.28 (1H, dt, J = 6.7, 3.4 Hz, 8""-H), 4.11 (1H, dd, J = 12.2, 6.7 Hz, 9""-H), 4.27 (1H, dd, J = 12.2,3.4 Hz, 9""-H), 7.77 (1H, t, J = 9.8 Hz, NH), 3.66 (3H, s, COOCH₃),1.98 (6H, OCOCH₃), 2.05 (6H, OCOCH₃), 1.69 (3H, s, NHCOCH₃), ¹³C-NMR (DMSO-*d*_{*6*}) δ: biflavonoid moiety; 163.6 (2-C), 103.8 (3-C), 181.9 (4-C), 161.1 (5-C), 98.1 (6-C), 165.2 (7-C), 92.5 (8-C), 157.3 (9-C), 104.7 (10-C), 122.2 (1'-C), 130.9 (2'-C), 120.5 (3'-C), 160.1 (4'-C), 111.6 (5'-C), 128.7 (6'-C), 164.3 (2"-C), 102.9 (3"-C), 182.4 (4"-C), 160.5 (5"-C), 102.0 (6"-C), 156.3 (7"-C), 108.2 (8"-C), 153.6 (9"-C), 106.4 (10"-C), 120.9 (1"'-C), 128.1 (2"'-C, 6"'-C), 115.9 (3"'-C, 5"'-C), 161.4 (4"'-C), 55.9 (7-OCH₃), 55.8 (4'-OCH₃), sialic acid moiety; 167.3 (1""-C), 100.1 (2""-C), 35.8 (3""-C), 68.4 (4""-C), 47.5 (5""-C), 73.1 (6""-C), 67.2 (7""-C), 68.6 (8""-C), 61.8 (9""-C), 53.4 (COOCH₃), 20.4 (OCOCH₃), 20.46 (2C, OCOCH₃), 20.52 (OCOCH₃), 22.5 (NHCOCH₃),169.2 (OCOCH₃), 169.3 (OCOCH₃), 169.5 (OCOCH₃), 170.0 (OCOCH₃),169.2 (NHCOCH₃).

### (S)-7,4'-di-O-methyl-7"-O-(1""-methyl-4"",7"",8""',9""-tetra-O-acetyl-N-acMetylneuraminyl)-4"'-O-(1""'-methyl-4""',7""',8""',9""'-tetra-O-acetyl-N-acetylneuraminyl)amentoflavone (31a)

Pale yellow powder; mp 165-169°C; UV λₘₐₓ (EtOH) (logε): 207 (4.81), 270 (4.71), 318 (4.60); IR νₘₐₓ (KBr) cm⁻¹: 3300, 2860, 1750, 1650, 1600, 1500, 1430, 1360, 1210, 1030, 840; positive FABMS *m*/*z* (rel. int. %): 1513 ([M+H]⁺, 6), 567 (30), 535 (8), 414 (7); ¹H-NMR (DMSO-*d*₆) δ: biflavonoid moiety; 6.99 (1H, s, 3-H), 6.39 (1H, d, J = 2.1 Hz, 6-H), 6.70 (1H, d, J = 2.1 Hz, 8-H), 8.14 (1H, d, J = 2.4 Hz, 2'-H), 7.40 (1H, d, J = 8.9 Hz, 5'-H), 8.26 (1H, dd, J = 8.9,2.4 Hz, 6'-H), 7.09 (1H, s, 3"-H), 6.97 (1H, s, 6"-H), 7.67 (2H, d, J = 9.2 Hz, 2"'-H, 6"'-H), 7.06 (2H, d, J = 9.2 Hz, 3"'-H, 5"'-H), 12.90 (1H, s, 5-OH), 13.09 (1H, s, 5"-OH), 3.85 (3H, s, 7-OCH₃), 3.84 (3H, s, 4'-OCH₃), sialic acid moiety; 1.88 (1H, m, 3""-H), 2.38 (1H, dd, J = 13.1, 4.9 Hz, 3""-H), 4.75 (1H, dt, J =10.7, 4.9 Hz, 4""-H), 3.93 (1H, m, 5""-H), 4.33 (1H, dd, J = 10.7, 1.8 Hz, 6""-H), 5.18 (1H, dd, J = 7. 0, 1. 8 Hz, 7""-H), 5.30 (1H, dt, J = 6.7, 3.4 Hz, 8""-H), 4.12 (1H, dd, J = 12.2, 6.7 Hz, 9""-H), 4.29 (1H, dd, J = 12.2,3.4 Hz, 9""-H), 2.04 (1 H, m, 3""'-H), 2.64 (1H, dd, J = 12.8, 4.3 Hz, 3""'-ED, 4.75 (1H, m, 4""'-H), 3.93 (1H, m, 5""'-H), 4.44 (1H, dd, J = 10. 7, 0.9 Hz, 6""' -H), 5.18 (1H, m, 7""'-H), 5.18 (1H, m, 8""'-H), 4.03 (1H, dd, J = 11.9, 4.7 Hz, 9""'-H), 4.12 (1H, m, 9""'-H), 7.80 (1H, t, J = 9.6 Hz, NH), 7.80 (1H, t, J = 9.6 Hz, NH), 3.66 (3H, s, 1""-COOCH₃), 3.53 (3H, s, 1""'-COOCH₃), 1.81 (3H, s, OCOCH₃), 1.93 (3H, s, OCOCH₃), 1.98 (3H, s, OCOCH₃), 1.99 (3H, s, OCOCH₃), 2.00 (3H, s, OCOCH₃), 2.04 (3H, s, OCOCH₃), 2.07 (3H, s, OCOCH₃), 2.07 (3H, s, OCOCH₃), 1.71 (3H, s, NHCOCH₃, 1.71 (3H, s, NHCOCH₃), ¹³C-NMR (DMSO-*d*_{*6*}) δ: biflavonoid moiety; 163.6 (2-C), 103.7 (3-C), 182.0 (4-C), 161.2 (5-C), 98.1 (6-C), 165.2 (7-C), 92.5 (8-C), 157.3 (9-C), 104.7 (10-C), 122.3 (1'-C), 130.8 (2'-C), 120.3 (3'-C), 160.0 (4'-C), 111.8 (5'-C), 128.7 (6'-C), 163.4 (2"-C), 104.6 (3"-C), 182.5 (4"-C), 161.0 (5"-C), 102.1 (6"-C), 156.5 (7"-C), 108.5 (8"-C), 153.7 (9"-C), 106.6 (10"-C), 125.3 (1"'-C), 127.9 (2"'-C, 6"'-C), 118.7 (3"'-C, 5"'-C), 156.7 (4"'-C), 55.9 (7-OCH₃), 55.8 (4'-OCH₃), sialic acid moiety; 167.2 (1""-C), 100.2 (2""-C), 35.8 (3""-C), 68.4 (4""-C), 47.5 (5""-C), 73.1 (6""-C), 67.2 (7""-C), 68.6 (8""-C), 61.8 (9""-C), 167.8 (1""'-C), 99.3 (2""'-C), 38.0 (3""'-C), 68.2 (4""'-C), 47.7 (5""'-C), 72.9 (6""'-C), 66.7 (7""'-C), 67.5 (8""'-C), 61.7 (9""'-C), 53.4 (1""-COOCH₃), 53.1 (1""'-COOCH₃), 20.38 (OCOCH₃), 20.41 (OCOCH₃), 20.50 (3C, OCOCH₃), 20.53 (OCOCH₃), 20.56 (OCOCH₃), 20.62 (OCOCH₃), 22.5 (2C, NHCOCH₃), 169.18 (OCOCH₃), 169.20 (OCOCH₃), 169.3 (OCOCH₃), 169.4 (OCOCH₃), 169.5 (OCOCH₃), 169.6 (OCOCH₃), 169.98 (OCOCH₃), 170.04 (OCOCH₃), 169.20 (NHCOCH₃), 169.20 (NHCOCH₃).

### (R)-7.4' -di-O-methyl-7" -O-(1 "" -methyl-4 "",7"" ,8"" ,9"" -tetra-O-acetyl-N-acetylneuraminyl)-4"'-O-(1""'-methyl-4""' ,7""' ,8""' ,9""'-tetra-O-acetyl-N acetylneuraminyl)amentoflavone (31b)

Pale yellow powder; mp 163-168°C; UV λₘₐₓ (EtOH) (logε): 210 (4.89), 270 (4.77), 317 (4.68); IR νₘₐₓ (KBr) cm⁻¹: 3400, 3000, 1760, 1660, 1610, 1510, 1440, 1380, 1270, 1230, 1040, 810; positive FABMS *m*/*z* (rel. int. %): 1513 ([M+H]⁺, 6), 567 (27), 414 (6); ¹H-NMR (DMSO-*d*_{*6*}) δ: biflavonoid moiety; 6.97 (1H, s, 3-H), 6.35 (1H, d, J = 2.1 Hz, 6-H), 6.76 (1H, d, J = 2.1 Hz, 8-H), 8.14 (1H, d, J = 2.4 Hz, 2'-H), 7.37 (1H, d, J = 8.9 Hz, 5'-H), 8.24 (1H, dd, J = 8.9,2.4 Hz, 6'-H), 7.07 (1H, s, 3"-H), 7.00 (1H, s, 6"-H), 7.63 (2H, d, J = 9.2 Hz, 2"'-H, 6"'-H), 7.04 (2H, d, J = 9.2 Hz, 3"'-H, 5"'-H), 12.90 (1H, s, 5-OH), 13.09 (5"-OH), 3.85 (3H, s, 7-OCH₃), 3.84 (3H, s, 4'-OCH₃), sialic acid moiety; 1.90 (1H, m, 3""-H), 2.36 (1H, dd, J = 12.8, 4.6 Hz, 3 ""-H), 4.73 (1H, m, 4""-H), 3.90 (1H, m, 5""-H), 4.25 (1H, m, 6""-H), 5.15 (1H, m, 7""-H), 5.08 (1H, m, 8""-H), 4.11 (1H, dd, J =12.1, 6.7 Hz, 9""-H), 4.28 (1H, dd, J = 12.1, 3.4 Hz, 9""-H), 2.01 (1H, m, 3""'-H), 2.65 (1H, dd, J = 12.5, 4.5 Hz, 3""'-H), 4.73 (1H, m, 4""' -H), 3.90 (1H, m, 5""'-H), 4.39 (1H, dd, J = 10.8, 1.4 Hz, 6""'-H), 5.15 (1H, m, 7""'-H), 5.28 (1H, dt, J = 6.7, 3.4 Hz, 8""'-H), 3.90 (1H, m, 9""' -H), 3.96 (1H, dd, J = 12.3, 4.7 Hz, 9""' -H), 7.77 (1H, t, J = 7.6 Hz, NH), 7.77 (1H, t, J = 7.6 Hz, NH), 3.60 (3H, s, 1""-COOCH₃), 3.59 (3H, s, 1""'-COOCH₃), 1.84 (3H, s, OCOCH₃), 1.92 (3H, s, OCOCH₃), 1.92 (3H, s, OCOCH₃), 1.94 (3H, s, OCOCH₃), 1.99 (3H, s, OCOCH₃), 2.06 (3H, s, OCOCH₃), 2.06 (3H, s, OCOCH₃), 2.07 (3H, s, OCOCH₃), 1.70 (3H, s, NHCOCH₃), 1.70 (3H, s, NHCOCH₃), ¹³C-NMR (DMSO-*d*_{*6*}) δ: biflavonoid moiety; 163.6 (2-C), 103.7 (3-C), 181.9 (4-C), 161.2 (5-C), 98.0 (6-C), 165.2 (7-C), 92.6 (8-C), 157.2 (9-C), 104.7 (10-C), 122.3 (1'-C), 130.7 (2'-C), 120.5 (3'-C), 160.3 (4'-C), 111.6 (5'-C), 128.5 (6'-C), 163.4 (2"-C), 104.7 (3"-C), 182.5 (4"-C), 160.4 (5"-C), 102.8 (6"-C), 156.4 (7"-C), 108.9 (8"-C), 153.8 (9"-C), 106.7 (10"-C), 125.5 (1"'-C), 127.9 (2"'-C, 6"'-C), 119.1 (3"'-C, 5"'-C), 156.6 (4"'-C), 56.0 (7-OCH₃), 55.8 (4'-OCH₃), sialic acid moiety; 166.9 (1""-C), 100.1 (2""-C), 35.8 (3""-C), 68.3 (4""-C), 47.5 (5""-C), 72.8 (6""-C), 66.5 (7""-C), 67.1 (8""-C), 61.2 (9""-C), 167.6 (1""'-C), 99.5 (2""'-C), 37.9 (3""'-C), 68.4 (4""'-C), 47.7 (5""'-C), 73.0 (6""'-C), 67.0 (7""'-C), 68.5 (8""'-C), 61.8 (9""'-C), 53.2 (1""-COOCH₃), 53.2 (1"'"-COOCH₃), 20.40 (3C, OCOCH₃), 20.44 (OCOCH₃), 20.49 (OCOCH₃), 20.52 (OCOCH₃), 20.58 (OCOCH₃), 20.62 (OCOCH₃), 22.5 (2C, NHCOCH₃), 169.0 (OCOCH₃), 169.18 (OCOCH₃), 169.19 (OCOCH₃), 169.3 (OCOCH₃), 169.5 (OCOCH₃), 169.6 (OCOCH₃), 169.8 (OCOCH₃), 170.0 (OCOCH₃), 169.19 (NHCOCH₃), 169.23 (NHCOCH₃).

### (R)-7,4',4"'-tri-O-methyl-7"-O-(1""-methyl-4"",7"",8"",9""-tetra-O-acetyl-N-acetylneuramin yl)amentoflavone (32a)

Pale yellow powder; mp 157-162°C; UV λₘₐₓ (EtOH) (logε): 205 (4.65), 270 (4.48), 330 (4.47); IR νₘₐₓ (KBr) cm⁻¹: 3300, 2875, 1750, 1650, 1600, 1505, 1430, 1370, 1260, 1210, 1030, 840; positive FABMS *m*/*z* (rel. int. %): 1054 ([M+H]⁺, 16), 581 (84), 549 (18), 414 (7); ¹H-NMR (DMSO-*d*_{*6*}) δ: biflavonoid moiety; 6.99 (1H, s, 3-H), 6.38 (1H, d, J=1.9 Hz, 6-H), 6.75 (1H, d, J = 1.9 Hz, 8-H), 8.15 (1H, d, J = 2.4 Hz, 2'-H), 7.38 (1H, d, J = 8.9 Hz, 5'-H), 8.25 (1H, dd, J = 8.9,2.4 Hz, 6'-H), 7.02 (1H, s, 3"-H), 6.98 (1H, s, 6"-H), 7.65 (2H, d, J = 8.9 Hz, 2"'-H, 6"'-H), 6.94 (2H, d, J = 8.9 Hz, 3"'-H, 5"'-H), 12.90 (1H, s, 5-OH), 13.11 (1H, s, 5"-OH), 3.83 (3H, s, 7-OCH₃), 3.81 (3H, s, 4'-OCH₃), 3.75 (3H, s, 4"'-OCH₃), sialic acid moiety; 1.93 (1H, dd, J = 12. 8, 11.9 Hz, 3""-H), 2.41 (1H, dd, J = 12.8,4.9 Hz, 3""-H), 4.73 (1H, dt, J = 11.9, 4.9 Hz, 4""-H), 3.92 (1H, m, 5""-H), 4.27 (1H, m, 6""-H), 5.17 (1H, dd, J = 6.7, 1. 8 Hz, 7""-H), 5.29 (1H, dt, J = 6.7, 3.2 Hz, 8""-H), 4.12 (1H, dd, J = 12.2, 6.7 Hz, 9""-H), 4.28 (1H, dd, J = 12.2, 3.2 Hz, 9""-H), 7.76 (1H, t, J = 9.5 Hz, NH), 3.62 (3H, s, COOCH₃), 1.85 (3H, s, OCOCH₃), 1.99 (3H, s, OCOCH₃), 2.06 (3H, s, OCOCH₃), 2.07 (3H, s, OCOCH₃), 1.71 (3H, s, NHCOCH₃), ¹³C-NMR (DMSO-*d*_{*6*}) δ: biflavonoid moiety; 163.5 (2-C), 103.8 (3-C), 181.9 (4-C), 161.1 (5-C), 98.0 (6-C), 165.2 (7-C), 92.7 (8-C), 157.3 (9-C), 104.7 (10-C), 122.2 (1'-C), 130.8 (2'-C), 120.6 (3'-C), 160.4 (4'-C), 111.6 (5'-C), 128.6 (6'-C), 163.9 (2"-C), 103.6 (3"-C), 182.4 (4"-C), 160.4 (5"-C), 102.5 (6"-C), 156.2 (7"-C), 108.6 (8"-C), 153.7 (9"-C), 106.5 (10"-C), 122.5 (1"'-C), 128.0 (2"'-C, 6"'-C), 114.6 (3"'-C, 5"'-C), 162.4 (4"'-C), 56.0 (7-OCH₃), 55.9 (4'-OCH₃), 55.5 (4"'-OCH₃), sialic acid moiety; 166.9 (1""-C), 100.1 (2""-C), 35.9 (3""-C), 68.4 (4""-C), 47.5 (5""-C), 73.0 (6""-C), 67.2 (7""-C), 68.5 (8""-C), 61.8 (9""-C), 53.1 (COOCH₃), 20.36 (2C, OCOCH₃), 20.44 (OCOCH₃), 20.5 (OCOCH₃), 22.5 (NHCOCH₃),169.2 (OCOCH₃), 169.3 (OCOCH₃), 169.5 (OCOCH₃), 170.0 (OCOCH₃), 169.2 (NHCOCH₃).

### (8)-7,4',4'"-tri-O-methyl-7" -O-(1''" -methyl-4'''' ,7"" ,8"" ,9"" -tetra-O-acetyl-N-acetylneuramin yl)amentoflavone (32b)

Pale yellow powder; mp 161-165°C; UV λₘₐₓ (EtOH) (logε): 205 (4.46), 268 (4.31), 325 (4.30); IR νₘₐₓ (KBr) cm⁻¹: 3230, 2860, 1750, 1660, 1610, 1510, 1440, 1370, 1210, 1030, 840; positive FABMS *m*/*z* (rel. int. %): 1054 ([M+H)⁺, 11), 581 (50), 549 (10); ¹H-NMR (DMSO-*d*₆) δ: biflavonoid moiety; 6.98 (1H, s, 3-H), 6.3 8 (1H, d, J = 1.9 Hz, 6-H), 6.70 (1H, d, J = 1.9 Hz, 8-H), 8.14 (1H, d, J = 2.4 Hz, 2'-H), 7.41 (1H, d, J = 8.9 Hz, 5'-H), 8.27 (1H, dd, J = 8.9, 2.4 Hz, 6'-H), 7.03 (1H, s, 3"-H), 6.97 (1H, s, 6"-H), 7.66 (2H, d, J = 8.9 Hz, 2"'-H, 6"'-H), 6.96 (2H, d, J = 8.9 Hz, 3"'-H, 5"'-H), 12.89 (1H, s, 5-OH), 13.13 (1H, s, 5"-OH), 3.84 (3H, s, 7-OCH₃), 3.82 (3H, s, 4'-OCH₃), 3.77 (3H, s, 4"'-OCH₃), sialic acid moiety; 1.90 (1H, m,3""-H), 2.41 (1H, dd, J = 12.8, 4.9 Hz, 3""-H), 4.75 (1H, dt, J = 10.7, 4.9 Hz, 4""-H), 3.93 (1H, m, 5""-H), 4.34 (1H, d, J = 11.6, 6""-H), 5.18 (1H, d, J = 6.7 Hz, 7""-H), 5.31 (1H, dt, J = 6.7, 3.1 Hz, 8""-H), 4.14 (1H, dd, J = 12.2, 6.7 Hz, 9""-H), 4.30 (1H, dd, J = 12.2, 3.1 Hz, 9""-H), 7.79 (1H, t, J = 9.5 Hz, NH), 3.69 (3H, s, COOCH₃), 1.83 (3H, s, OCOCH₃), 2.01 (3H, s, OCOCH₃), 2.07 (3H, s, OCOCH₃), 2.07 (3H, s, OCOCH₃), 1.72 (3H, s, NHCOCH₃), ¹³C-NMR (DMSO-*d*_{*6*}) δ: biflavonoid moiety; 163.5 (2-C), 103.8 (3-C), 181.9 (4-C), 161.1 (5-C), 98.1 (6-C), 165.2 (7-C), 92.5 (8-C), 157.3 (9-C), 104.7 (10-C), 122.3 (1'-C), 130.8 (2'-C), 120.5 (3'-C), 160.1 (4'-C), 111.6 (5'-C), 128.7 (6'-C), 163.9 (2"-C), 103.6 (3"-C), 182.4 (4"-C), 160.5 (5"-C), 102.2 (6"-C), 156.3 (7"-C), 108.3 (8"-C), 153.6 (9"-C), 106.5 (10"-C), 122.5 (1"'-C), 128.0 (2"'-C, 6"'-C), 114.6 (3"'-C, 5"'-C), 162.5 (4"'-C), 55.9 (7-OCH₃), 55.8 (4'-OCH₃), 55.5 (4"'-OCH₃), sialic acid moiety; 167.2 (1""-C), 100.1 (2""-C), 35.8 (3""-C), 68.4 (4""-C), 47.5 (5""-C), 73.1 (6""-C), 67.2 (7""-C), 68.6 (8""-C), 61.9 (9""-C), 53.3 (COOCH₃), 20.4 (2C, OCOCH₃), 20.45 (OCOCH₃), 20.51 (OCOCH₃), 22.5 (NHCOCH₃),169.2 (OCOCH₃), 169.3 (OCOCH₃), 169.5 (OCOCH₃), 170.0 (OCOCH₃), 169.2 (NHCOCH₃).

### 3 Deprotection of the acetyl-protected sialyl biflavonoids (Fig. 4)

Each of **29a** (100.2 mg, 0.095 mmol), **29b** (100.0 mg, 0.095 mmol), **32a** (100.0 mg, 0.095 mmol), and **32b** (100.0 mg, 0.095 mmol) was dissolved in 5 ml pure MeOH, to which sodium methoxide (NaOMe) was added to reach pH>8 (confirmed using a pH-test paper), and the solution was stirred at room temperature for two hours, followed by the addition of 5 ml of H₂O and stirring for additional 24 hours. After completion of the reaction, the MeOH solution was dried in vacuo, and then a new 5 ml H₂O, followed by Dowex 50W-X2 (2.0 g), was added, and the mixture was stirred for 20 minutes. After that, Dowex 50W-X2 was removed by vacuum filtration, and the H₂O was dried in vacuo to yield sialyl flavonoids **33a** (75.1 mg, 90.6%), **33b** (81.6 mg, 98.7%), **36a** (84.6 mg, 100.0%), and **36b** (84.9 mg, 100.0%), respectively.

Meanwhile, each of **30a** (57.5 mg, 0.055 mmol), **30b** (65.3 mg, 0.063 mmol), **31a** (100.0 mg, 0.066 mmol), and **31b** (100.0 mg, 0.066 mmol) was dissolved in 5 ml of pure MeOH, to which sodium methoxide (NaOMe) was added to reach pH>8 (confirmed using a pH-test paper), and the solution was stirred at room temperature for two to nine hours, followed by addition of 5 ml of H₂O and stirring for additional 24 hours. After the reaction, the solution was neutralized with 3.5% HCl. After the solvent was dried in vacuo, the salt was removed using HP-20, and then the product was purified by HPLC (reverse phase, SENSHU PAK ODS-4251-SH, 10φx 250 mm, 80% MeOH) to yield sialyl biflavonoids **34a** (45.7 mg, 96.4%), **34b** (41.1 mg, 76.3%), **35a** (52.0 mg, 68.5%), and **35b** (60.8 mg, 80.1%), respectively.

### (R)-4"'-O-(N-acetylneuraminyl)-7,4',7"-tri-O-methylamentoflavone (33a)

Pale yellow powder; mp 191°C (dec.); UV λₘₐₓ (MeOH) (logs): 210 (4.92), 270 (4.83), 325 (4.76); IR λₘₐₓ (KBr) cm⁻¹: 3350, 2950, 1660, 1610, 1510, 1440, 1380, 1340, 1260, 1210, 1160,1130,1030, 840; positive FABMS *m*/*z* (rel. int. %): 872 ([M+H]⁺, 1), 581 (10); ¹H-NMR (DMSO-*d*_{*6*}) δ: biflavonoid moiety; 6.96 (1H, s, 3-H), 6.30 (1H, bs, 6-H), 6.74 (1H, s, 8-H), 8.04 (1H, d, J = 1.8 Hz, 2'-H), 7.41 (1H, d, J = 8.9 Hz, 5'-H), 8.20 (1H, m, 6'-H), 6.92 (1H, s, 3"-H), 6.65 (1H, s, 6"-H), 7.49 (2H, d, J = 8.9 Hz, 2"'-H, 6"'-H), 7.19 (2H, d, J = 8.9 Hz, 3"'-H, 5"'-H), 12.89 (1H, s, 5-0H), 13.20 (1H, s, 5"-OH), 3.78 (3H, s, 7-OCH₃), 3.75 (3H, s, 4'-OCH₃), 3.80 (3H, s, 7"-OCH₃), sialic acid moiety; 1.56 (1H, t, J = 11.9 Hz, 3-H""), 2.83 (1H, dd, J = 11.9, 4.9 Hz, 3-H""), 3.58 (1H, m, 4-H""), 3.3 8 (1H, m, 5-H""), 3.74 (1H, m, 6-H""), 3.32 (1H, m, 7-H""), 3.61 (1H, m, 8-H""), 3.43 (1H, m, 9-H""), 3.64 (1H, m, 9-H""), 8.35 (1H, bs, NH), 5.27 (1H, bs, OH), 4.70 (1H, d, J = 3.1 Hz, OH), 4.30 (1H, bs, OH), 1.89 (3H, s, NHCOCH₃), ¹³C-NMR (DMSO-*d*_{*6*}) δ: biflavonoid moiety; 163.5 (2-C), 103.9 (3-C), 181.9 (4-C), 161.1 (5-C), 98.1 (6-C), 165.2 (7-C), 92.7 (8-C), 157.3 (9-C), 104.7 (10-C), 122.4 (1'-C), 130.8 (2'-C), 121.3 (3'-C), 160.5 (4'-C), 111.8 (5'-C), 128.4 (6'-C), 163.9 (2"-C), 102.5 (3"-C), 182.2 (4"-C), 161.5 (5"-C), 95.5 (6"-C), 165.5 (7"-C), 104.5 (8"-C), 153.4 (9"-C), 104.0 (10"-C), 121.1 (1"'-C), 128.1 (2"'-C, 6"'-C), 115.8 (3"'-C, 5"'-C), 161.2 (4"'-C), 56.0 (7-OCH₃), 56.0 (4'-OCH₃), 56.5 (7"-OCH₃), sialic acid moiety; 168.8 (1""-C), 101.9 (2""-C), 42.6 (3""-C), 66.8 (4""-C), 52.9 (5""-C), 73.7 (6""-C), 69.0 (7""-C), 71.6 (8""-C), 63.4 (9""-C), 22.4 (NHCOCH₃), 172.3 (NHCOCH₃).

### (S)-4"'-O-(N-acetylneuraminyl)-7,4',7"-tri-O-methylamentoflavone (33b)

Pale yellow powder; mp 184°C(dec.); UV λₘₐₓ (MeOH) (logs): 207 (4.86), 270 (4.74), 325 (4.67); IR νₘₐₓ (KBr) cm⁻¹: 3400, 2950, 1660,1600,1500,1440,1380, 1340, 1260, 1200, 1160,1120,1020, 840; positive FABMS *m*/*z* (rel. int. %): 872 ([M+H]⁺, 1), 581 (12); ¹H-NMR (DMSO-*d*_{*6*}) δ: biflavonoid moiety; 6.93 (1H, s, 3-H), 6.31 (1H, bs, 6-H), 6.73 (1H, bs, 8-H), 8.05 (1H, d, J = 2.4 Hz, 2'-H), 7.39 (1H, d, J = 8.9 Hz, 5'-H), 8.20 (1H, m, 6'-H), 6.92 (1H, s, 3"-H), 6.65 (1H, bs, 6"-H), 7.51 (2H, d, J = 8.9 Hz, 2"'-H, 6"'-H), 7.18 (2H, d, J = 8.9 Hz, 3"'-H, 5"'-H), 12.89 (1H, s, 5-OH), 13.16 (1H, s, 5"-OH), 3.78 (3H, s, 7-OCH₃), 3.78 (3H, s, 4'-OCH₃), 3.80 (3H, s, 7"-OCH₃), sialic acid moiety; 1.63 (1H, t, J = 11.9 Hz, 3-H""), 2.77 (1H, dd, J = 11.9,4.6 Hz, 3-H""), 3.61 (1H, m, 4-H""), 3.52 (1H, m, 5-H""), 3.72 (1H, m, 6-H""), 3.32 (1H, m, 7-H""), 3.59 (1H, m, 8-H""), 3.42 (1H, m, 9-H""), 3.63 (1H, m, 9-H""), 8.20 (1H, bs, NH), 5.18 (1H, bs, OH), 4.67 (1H, bs, OH), 4.23 (1H, bs, OH), 1.89 (3H, s, NHCOCH3), ¹³C-NMR (DMSO-*d*_{*6*}) δ: biflavonoid moiety; 163.^{a)} (2-C), 104.0^{b)} (3-C), 181.8 (4-C), 161.1 (5-C), 97.9 (6-C), 165.0 (7-C), 92.6 (8-C), 157.2 (9-C), 104.7^{c)} (10-C), 122.5 (1'-C), 130.7 (2'-C), 121.1 (3'-C), 160.3 (4'-C), 111.7 (5'-C), 128.4 (6'-C), 163.4^{a)} (2"-C), 103.8^{b)} (3"-C), 182.2 (4"-C), 161.4 (5"-C), 95.5 (6"-C), 162.4 (7"-C), 104.5^{c)} (8"-C), 153.4 (9"-C), 104.0^{b)} (10"-C), 124.3 (1"'-C), 126.9 (2"'-C, 6"'-C), 120.4 (3"'-C, 5"'-C), 158.2 (4"'-C), 55.9^{d)} (7-OCH₃), 55.9^{d)} (4'-OCH₃), 56.3 ^{d)} (7"-OCH₃), sialic acid moiety; 168.9 (1""-C), 101.6 (2""-C), 41.8 (3""-C), 66.6 (4""-C), 52.5 (5""-C), 73.9 (6""-C), 68.7 (7""-C), 71.4 (8""-C), 63.1 (9""-C), 22.5 (NHCOCH₃), 172.1 (NHCOCH₃). a), b), c), d): may be interchanged.

### (R)-7" -O-(N-acetylneuraminyl)-7.4' -di-O-methylamentoflavone (34a)

Pale yellow powder; mp 224°C (dec); UV λₘₐₓ (MeOH) (logε): 205 (4.58), 268 (4.44), 328 (4.42); IR νₘₐₓ (KBr) cm⁻¹: 3400, 2950, 1650, 1600, 1500, 1440, 1370, 1250, 1160, 1110, 1020, 840; positive FABMS m/z (rel. int. %): 857 ([M+H]⁺, 1), 567 (2); ¹H-NMR (DMSO-*d*_{*6*}) δ: biflavonoid moiety; 6.99 (1H, s, 3-H), 6.34 (1H, d, J = 2.1 Hz, 6-H), 6.76 (1H, d, J = 2.1 Hz, 8-H), 8.05 (1H, d, J = 2.1 Hz, 2'-H), 7.3 5 (1H, d, J = 8.9 Hz, 5'-H), 8.18 (1H, dd, J = 8.9, 2.1 Hz, 6'-H), 6.80 (1H, s, 3"-H), 7.34 (1H, s, 6"-H), 7.50 (2H, d, J = 8.9 Hz, 2"'-H, 6"'-H), 6.71 (2H, d, J = 8.9 Hz, 3"'-H, 5"'-H), 12.97 (1H, bs, 5-OH), 12.97 (1H, bs, 5"-OH), 3.83 (3H, s, 7-OCH₃), 3.79 (3H, s, 4'-OCH₃), sialic acid moiety; 1.46 (1H, t, J = 11.9 Hz, 3-H""), 2.75 (1H, dd, J = 11.9, 4.9 Hz, 3-H""), 3.59 (1H, m, 4-H""), 3.40 (1H, m, 5-H""), 3.71 (1H, m, 6-H""), 3.31 (1H, m, 7-H""), 3.67 (1H, m, 8-H""), 3.42 (1H, m, 9-H""), 3.61 (1H, m, 9-H""), 8.30 (1H, bs, NH), 6.25 (1H, bs, OH), 5.14 (1H, bs, OH), 4.72 (1H, s, OH), 4.14 (1H, bs, OH), 1.89 (3H, s, NHCOCH₃), ¹³C-NMR (DMSO-*d*_{*6*}) δ: biflavonoid moiety; 163.9 (2-C), 103.9 (3-C), 181.9 (4-C), 161.0 (5-C), 98.3 (6-C), 165.1 (7-C), 92.4 (8-C), 157.4 (9-C), 104.8 (10-C), 122.1 (1'-C), 131.0 (2'-C), 121.7 (3'-C), 160.5 (4'-C), 111.6 (5'-C), 128.2 (6'-C), 163.8 (2"-C), 102.3 (3"-C), 182.1 (4"-C), 160.0 (5"-C), 102.0 (6"-C), 158.8 (7"-C), 106.2 (8"-C), 153.2 (9"-C), 104.5 (10"-C), 120.6 (1"'-C), 128.0 (2"'-C, 6"'-C), 115.9 (3"'-C, 5"'-C), 161.8 (4"'-C), 56.0 (7-OCH₃), 55.9 (4'-OCH₃), sialic acid moiety; 168.1 (1""-C), 102.2 (2""-C), 41.7 (3""-C), 66.9 (4""-C), 52.9 (5""-C), 73.6 (6""-C), 68.8 (7""-C), 71.1 (8""-C), 63.0 (9""-C), 22.4 (NHCOCH₃), 172.3 (NHCOCH₃).

### (S)-7"-O-(N-acetylneuraminyl)-7,4'-di-O-methylamentoflavone (34b)

Pale yellow powder; mp 209°C (dec); UV λₘₐₓ (MeOH) (log ε): 208 (4.62), 268 (4.49), 328 (4.47); IR νₘₐₓ (KBr) cm⁻¹: 3400, 2950, 1650, 1610, 1500, 1440, 1370, 1340, 1280, 1260, 1240, 1160,1110,1020, 950, 840; positive FABMS *m*/*z* (rel. int. %): 857 ([M+H]⁺, 1), 567 (2); ¹H-NMR (DMSO-d6) δ: biflavonoid moiety; 7.06 (1H, s, 3-H), 6.36 (1H, d, J = 1.8 Hz, 6-H), 6.91 (1H, d, J = 1.8 Hz, 8-H), 8.67 (1H, d, J = 2.1 Hz, 2'-H), 7.35 (1H, d, J = 8.9 Hz, 5'-H), 8.18 (1H, dd, J = 8.9, 2.1 Hz, 6'-H), 6.77 (1H, s, 3"-H), 7.10 (1H, s, 6"-H), 7.48 (2H, d, J = 8.9 Hz, 2"'-H, 6"'-H), 6.73 (2H, d, J = 8.9 Hz, 3"'-H, 5"'-H), 12.99 (1H, bs, 5-OH), 12.99 (1H, bs, 5"-OH), 3.85 (3H, s, 7-OCH₃), 3.77 (3H, s, 4'-OCH₃), sialic acid moiety; 1.33 (1H, t, J = 11.9 Hz, 3""-H), 2.80 (1H, dd, J = 11.9, 4.6 Hz, 3""-H), 3.51 (1H, m, 4""-H), 3.40 (1H, m, 5""-H), 3.88 (1H, m, 6""-H), 3.34 (1H, m, 7""-H), 3.72 (1H, m, 8""-H), 3.45 (1H, m, 9""-H), 3.65 (1H, m, 9""-H), 8.18 (1H, bs, NH), 6.41 (1H, bs, OH), 4.81 (1H, bs, OH), 4.62 (1H, s, OH), 4.21 (1H, bs, OH), 1.89 (3H, s, NHCOCH₃), ¹³C-NMR (DMSO-*d*_{*6*}) δ: biflavonoid moiety; 163.9^{a)} (2-C), 103.4^{b)} (3-C), 182.0 (4-C), 161.1 (5-C), 97.8 (6-C), 165.1 (7-C), 92.9 (8-C), 157.3 (9-C), 104.7 (10-C), 122.1 (1'-C), 132.4 (2'-C), 121.4 (3'-C), 159.7 (4'-C), 111.1 (5'-C), 127.7 (6'-C), 163.9^{a)} (2"-C), 102.1 (3"-C), 182.1 (4"-C), 160.1 (5"-C), 103.1 (6"-C), 159.0 (7"-C), 106.7 (8"-C), 152.9 (9"-C), 105.1 (10"-C), 120.0 (1"'-C), 128.0 (2"'-C, 6"'-C), 116.1 (3"'-C, 5"'-C), 162.3 (4"'-C), 56.0 (7-OCH₃), 55.7 (4'-OCH₃), sialic acid moiety; 167.8 (1""-C), 103.5^{b)} (2""-C), 42.6 (3""-C), 67.0 (4""-C), 53.0 (5""-C), 73.7 (6""-C), 68.7 (7""-C), 71.5 (8""-C), 63.1 (9""-C), 22.5 (NHCOCH₃), 172.2 (NHCOCH₃). a), b): may be interchanged.

### (S)-7",4'"-di-O-(N-acetylneuraminyl)-7,4'-di-O-methylamentoflavone (35a)

Pale yellow powder; mp 188°C (dec); UV λₘₐₓ (MeOH) (logε): 207 (4.66), 270 (4.50), 325 (4.46); IR νₘₐₓ (KBr) cm-1: 3400, 2930,1650,1600,1500,1440, 1370, 1340, 1280, 1250, 1160, 1110, 1020, 840; positive FABMS *m*/*z* (rel. int. %): 1149 ([M+H]⁺, 1), 567 (1); ¹H-NMR (DMSO-*d*_{*6*}) δ: biflavonoid moiety; 7.07 (1H, s, 3-H), 6.35 (1H, d, J = 2.1 Hz, 6-H), 6.93 (1H, d, J = 2.1 Hz, 8-H), 8.70 (1H, d, J = 2.1 Hz, 2'-H), 7.41 (1H, d, J = 8.9 Hz, 5'-H), 8.21 (1H, dd, J = 2.1, 8.9 Hz, 6'-H), 6.93 (1H, s, 3"-H), 7.10 (1H, s, 6"-H), 7.55 (2H, d, J = 8.9 Hz, 2"'-H, 6"'-H), 7.24 (2H, d, J = 8.9 Hz, 3"'-H, 5"'-H), 12.93 (1H, s, 5-OH), 12.93 (1H, s, 5"-OH), 3.86 (3H, s, 7-OCH₃), 3.76 (3H, s, 4'-OCH₃), sialic acid moiety; 1.58 (1H, t, J = 11.6 Hz, 3""-H), 2.84 (1H, dd, J = 12.8,4.3 Hz, 3""-H), 3.57 (1H, m, 4""-H), 3.38 (1H, m, 5""-H), 3.91 (1H, m, 6""-H), 3.29 (1H, m, 7""-H), 3.74 (1H, m, 8""-H), 3.68 (1H, m, 9""-H), 3.47 (1H, m, 9""-H), 1.3 5 (1H, t, J = 11.6 Hz, 3""'-H), 2.84 (1H, dd, J = 12.8,4.3 Hz, 3""'-H), 3.57 (1H, m, 4""'-H), 3.34 (1H, m, 5""'-H), 3.79 (1H, m, 6""'-H), 3.29 (1H, m, 7""'-H), 3.65 (1H, m, 8""'-H), 3.68 (1H, m, 9""'-H), 3.47 (1H, m, 9""'-H), 8.31 (1H, bs, NH), 8.25 (1H, bs, NH), 6.31 (1H, bs, OH), 6.18 (1H, bs, OH), 5.30 (1H, bs, OH), 4.92 (1H, bs, OH), 4.72 (1H, bs, OH), 4.65 (1H, bs, OH), 4.38 (1H, t, J = 5.5 Hz, OH), 4.26 (1H, t, J = 5.8 Hz, OH), 1.90 (3H, s, NHCOCH₃), 1.86 (3H, s, NHCOCH₃), ¹³C-NMR (DMSO-*d*_{*6*}) δ: biflavonoid moiety; 163.9 (2-C), 103.5^{a)} (3-C), 182.1 (4-C), 161.0 (5-C), 97.9 (6-C), 165.1 (7-C), 92.8 (8-C), 157.4 (9-C), 104.8 (10-C), 122.2 (1'-C), 132.5 (2'-C), 121.2 (3'-C), 160.0 (4'-C), 111.2 (5'-C), 127.9 (6'-C), 163.3 (2"-C), 103.1 (3"-C), 182.3 (4"-C), 159.8 (5"-C), 103.1 (6"-C), 159.1^{b)} (7"-C), 106.8 (8"-C), 153.0 (9"-C), 105.2 (10"-C), 123.0 (1"'-C), 126.9 (2"'-C, 6"'-C), 119.2 (3"'-C, 5"'-C), 159.0^{b)} (4"'-C), 56.0 (7-OCH₃), 55.7 (4'-OCH₃), sialic acid moiety; 168.5 (1""-C), 101.8 (2""-C), 42.8 (3""-C), 66.9 (4""-C), 53.1 (5""-C), 73.8 (6""-C), 69.1 (7""-C), 71.7 (8""-C), 63.4 (9""-C), 169.0 (1""'-C), 103.5^{a)} (2""'-C), 42.8 (3""'-C), 66.8 (4""'-C), 53.0 (5""'-C), 73.7 (6""'-C), 68.7 (7""'-C), 71.6 (8""'-C), 63.0 (9""'-C), 22.5 (2C, NHCOCH₃), 172.4 (NHCOCH₃), 172.3 (NHCOCH₃). a), b): may be interchanged.

### (R)-7",4"'-di-O-(N-acetylneuraminyl)-7,4'-di-O-methylamentoflavone (35b)

Pale yellow powder; 194°C (dec); UV λₘₐₓ (EtOH) (log ε): 207 (4.73), 270 (4.59), 325 (4.55); IR νₘₐₓ (KBr) cm⁻¹: 3400, 2940, 1650, 1610, 1500, 1440, 1370, 1340, 1280,1260, 1240, 1160,1110,1020, 950, 840; positive FABMS *m*/*z* (rel. int. %): 1149 ([M+H]⁺, 1), 567 (1); ¹H-NMR (DMSO-*d*_{*6*}) δ: biflavonoid moiety; 6.96 (1H, s, 3-H), 6.33 (1H, d, J = 2.1 Hz, 6-H), 6.76 (1H, d, J = 2.1 Hz, 8-H), 7.99 (1H, d, J = 2.1 Hz, 2'-H), 7.3 8 (1H, d, J = 8.9 Hz, 5'-H), 8.17 (1H, dd, J = 8.9,2.1 Hz, 6'-H), 6.91 (1H, s, 3"-H), 7.34 (1H, s, 6"-H), 7.51 (2H, d, J = 8.9 Hz, 2"'-H, 6"'-H), 7.18 (2H, d, J = 8.9 Hz, 3"'-H, 5'"-H), 12.93 (1H, s, 5-OH), 12.90 (1H, s, 5"-OH), 3.83 (3H, s, 7-OCH₃), 3.80 (3H, s, 4'-OCH₃), sialic acid moiety; 1.54 (1H, t, J = 11.6 Hz, 3""-H), 2.80 (1H, dd, J = 12.8, 4.3 Hz, 3""-H), 3.S 8 (1H, m, 4""-H), 3.42 (1H, m, 5""-H), 3.69 (1H, m, 6""-H), 3.30 (1H, m, 7""-H), 3.56 (1H, m, 8""-H), 3.64 (1H, m, 9""-H), 3.42 (1H, m, 9""-H), 1.54 (1H, t, J = 11.6 Hz, 3""'-H), 2.80 (1H, dd, J = 12.8,4.3 Hz, 3""'-H), 3.58 (1H, m, 4""'-H), 3.42 (1H, m, 5""'-H), 3.69 (1H, m, 6""'-H), 3.30 (1H, m, 7""'-H), 3.66 (1H, m, 8""'-H), 3.64 (1H, m, 9""'-H), 3.42 (1H, m, 9""' -H), 8.41 (1H, bs, NH), 8.41 (1H, bs, NH), 6.14 (1H, bs, OH), 5.33 (1H, bs, OH), 4.78 (1H, bs, OH), 4.72 (1H, bs, OH), 4.28 (1H, bs, OH), 1.88 (3H, s, NHCOCH₃), 1.87 (3H, s, NHCOCH3), ¹³C-NMR (DMSO-*d*_{*6*}) δ: biflavonoid moiety; 164.0 (2-C), 104.0 (3-C), 181.9 (4-C), 161.0 (5-C), 98.4 (6-C), 165.2 (7-C), 92.3 (8-C), 157.5 (9-C), 104.8 (10-C), 122.3 (1'-C), 131.0 (2'-C), 121.5 (3'-C), 160.4 (4'-C), 111.6 (5'-C), 128.3 (6'-C), 163.4 (2"-C), 103.4 (3"-C), 182.3 (4"-C), 160.0 (5"-C), 102.2 (6"-C), 158.7 (7"-C), 106.3 (8"-C), 153.3 (9"-C), 104.7 (10"-C), 123.8 (1"'-C), 126.9 (2"'-C, 6"'-C), 120.2 (3"'-C, 5"'-C), 158.9 (4"'-C), 55.9 (7-OCH₃), 56.0 (4'-OCH₃), sialic acid moiety; 168.7 (1""-C), 102.2 (2""-C), 41.6 (3""-C), 66.7 (4""-C), 53.0 (5""-C), 73.7 (6""-C), 68.7 (7""-C), 71.2 (8""-C), 62.9 (9""-C), 168.9 (1""'-C), 102.3 (2""'-C), 42.4 (3""'-C), 66.8 (4""'-C), 53.0 (5""'-C), 73.8 (6""'-C), 68.7 (7""'-C), 71.6 (8""'-C), 63.1 (9""'-C), 22.4 (NHCOCH₃), 22.4 (NHCOCH₃), 172.3 (NHCOCH₃), 172.4 (NHCOCH₃).

### (R)-7"-O-(N-acetylneuraminyl)-7.4',4'"-tri-O-methylamentoflavone (36a)

Pale yellow powder; mp 206°C (dec.); UV λₘₐₓ (MeOH) (logs): 206 (4.88), 269 (4.80), 325 (4.73); IR νₘₐₓ (KBr) cm⁻¹: 3400, 2950, 1660, 1600, 1500, 1440, 1370, 1350, 1260, 1180, 1160,1120,1030, 840; positive FABMS *m*/*z* (rel. int. %): 872 ([M+H]⁺, 1), 581 (12); ¹H-NMR (DMSO-*d*_{*6*}) δ: biflavonoid moiety; 6.99 (1H, s, 3-H), 6.35 (1H, bs, 6-H), 6.75 (1H, bs, 8-H), 8.08 (1H, bs, 2'-H), 7.3 7 (1H, d, J = 8.9 Hz, 5'-H), 8.22 (1H, d, J = 8.9 Hz, 6' -H), 6.97 (1H, s, 3"-H), 7.18 (1H, s, 6"-H), 7.64 (2H, d, J = 8.5 Hz, 2"'-H, 6"'-H), 6.95 (2H, d, J = 8.5 Hz, 3"'-H, 5"'-H), 12.92 (1H, s, 5-OH), 13.01 (1H, s, 5"-OH), 3.83 (3H, s, 7-OCH₃), 3.80 (3H, s, 4'-OCH₃), 3.76 (3H, s, 4"'-OCH₃), sialic acid moiety; 1.67 (1H, t, J = 11.9 Hz, 3""-H), 2.57 (1H, m, 3""-H), 3.65 (1H, m, 4""-H), 3.61 (1H, m, 5""-H), 3.80 (1H, m, 6""-H), 3.36 (1H, m, 7""-H), 3.65 (1H, m, 8""-H), 3.65 (1H, m, 9""-H), 3.45 (1H, m, 9""-H), 8.10 (1H, d, J = 6.4 Hz, NH), 5.08 (1H, bs, OH), 4.68 (1H, bs, OH), 4.24 (1H, bs, OH), 1.89 (3H, s, NHCOCH₃), ¹³C-NMR (DMSO-*d*₆) δ: biflavonoid moiety; 163.6^{a)} (2-C), 103.9 (3-C), 181.9 (4-C), 161.1 (5-C), 98.2 (6-C), 165.2 (7-C), 92.5 (8-C), 157.4 (9-C), 104.8 (10-C), 122.2 (1'-C), 130.9 (2'-C), 121.1 (3'-C), 160.1 (4'-C), 111.6 (5'-C), 128.4 (6'-C), 163.7^{a)} (2"-C), 103.4 (3"-C), 182.3 (4"-C), 160.4 (5"-C), 102.2 (6"-C), 157.8 (7"-C), 107.2 (8"-C), 153.5 (9"-C), 105.4 (10"-C), 122.6 (1"'-C), 127.9 (2"'-C, 6"'-C), 114.5 (3"'-C, 5"'-C), 162.3 (4"'-C), 56.0 (7-OCH₃), 55.9 (4'-OCH₃), 55.5 (4"'-OCH₃), sialic acid moiety; 168.6 (1""-C), 101.1 (2""-C), 40.4 (3""-C), 66.3 (4""-C), 52.3 (5""-C), 74.4 (6""-C), 68.7 (7""-C), 71.0 (8""-C), 63.2 (9""-C), 22.6 (NHCOCH₃), 171.9 (NHCOCH₃). a): may be interchanged.

### (S')-7" -O-(N-acetylneuraminyl)-7,4'4'", -tri -O-methylamentoflavone (36b)

Pale yellow powder; mp 200°C (dec.); UV λₘₐₓ (MeOH) (logε): 206 (4.86), 270 (4.78), 325 (4.71); IR _{νmax} (KBr) cm⁻¹: 3400, 2950, 1650, 1610, 1510, 1440, 1370, 1340, 1260, 1180, 1160, 1110, 1030, 840; positive FABMS *m*/*z* (rel. int. %): 872 ([M+H]⁺, 1), 581 (15); ¹H-NMR (DMSO-*d*₆) δ: biflavonoid moiety; 7.02 (1H, s, 3-H), 6.30 (1H, d, J = 1.8 Hz, 6-H), 6.87 (1H, d, J =1.8 Hz, 8-H), 8.57 (1H, bs, 2'-H), 7.36 (1H, d, J = 8.9 Hz, 5'-H), 8.18 (1H, d, J = 8.9 Hz, 6'-H), 6.93 (1H, s, 3"-H), 7.11 (1H, s, 6"-H), 7.65 (2H, d, J = 8.9 Hz, 2"'-H, 6"'-H), 6.98 (2H, d, J = 8.9 Hz, 3"'-H, 5"'-H), 12.90 (1H, s, 5-OH), 12.93 (1H, s, 5"-OH), 3.86 (3H, s, 7-OCH₃), 3.78 (3H, s, 4'-OCH₃), 3.78 (3H, s, 4"'-OCH₃), sialic acid moiety; 1.38 (1H, t, J = 11.9 Hz, 3""-H), 2.77 (1H, m, 3""-H), 3.55 (1H, m, 4""-H), 3.40 (1H, m, 5""-H), 3.89 (1H, d, J = 5.2 Hz, 6""-H), 3.34 (1H, m, 7""-H), 3.72 (1H, m, 8""-H), 3.45 (1H, m, 9""-H), 3.65 (1H, m, 9""-H), 8.21 (1H, bs, NH), 4.95 (1H, bs, OH), 4.64 (1H, d, J = 3.7 Hz, OH), 4.26 (1H, bs, OH), 1.88 (3H, s, NHCOCH₃), ¹³C-NMR (DMSO-d₆) δ: biflavonoid moiety; 163.8 (2-C), 103.5 (3-C), 182.0 (4-C), 161.1 (5-C), 97.9 (6-C), 165.1 (7-C), 92.8 (8-C), 157.3 (9-C), 104.7 (10-C), 122.2 (1'-C), 132.2 (2'-C), 121.2 (3'-C), 160.1 (4'-C), 111.3 (5'-C), 127.9 (6'-C), 163.4 (2"-C), 103.3 (3"-C), 182.3 (4"-C), 159.9 (5"-C), 102.9^{a)} (6"-C), 159.0 (7"-C), 106.8 (8"-C), 153.1 (9"-C), 105.2 (10"-C), 122.7 (1"'-C), 127.9 (2"'-C, 6"'-C), 114.6 (3"'-C, 5"'-C), 162.3 (4"'-C), 55.9 (7-OCH₃), 55.8 (4'-OCH₃), 55.5 (4"'-OCH₃), sialic acid moiety; 168.4 (1""-C), 103.1^{a)} (2""-C), 42.4 (3""-C), 66.8 (4""-C), 52.9 (5""-C), 73.9 (6""-C), 68.7 (7""-C), 71.5 (8""-C), 63.1 (9""-C), 22.5 (NHCOCH₃), 172.2 (NHCOCH₃). a): may be interchanged.

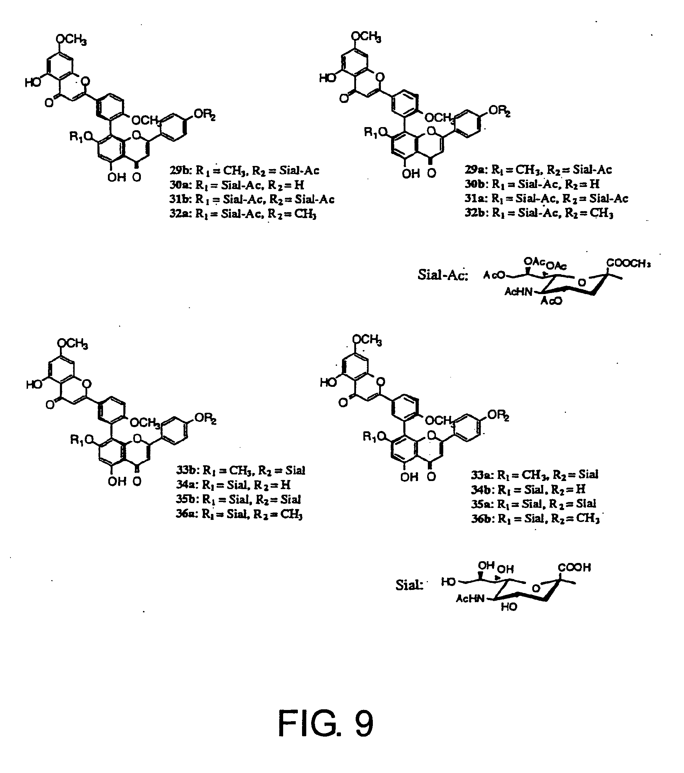

Fig. 9 shows the structure of the synthesized sialyl biflavonoids.

### [Example 3] Inhibitory activity against the influenza virus sialidase

The four biflavonoids (**19**, **20**, **21**, and **22**) isolated from plants, and the 19 biflavonoid-sialic acid conjugates prepared by organic synthesis [Gin-Neu-1-(R) **(34a),** Gin-Neu-2-(S) **(35a),** Gin=Neu-1-(S) **(34b),** Gin-Neu-2-(R) **(35b),** Gin-NeuAc-1 **(30a),** Gin-NeuAc-2 **(31a),** Gin-NeuAc-3 **(30b),** Gin-NeuAc-4 **(31b),** Gin-NeuAc-di(Mix) (mixture of **31a** and **31b**), TN1-Neu-1 **(33a),** TN1-Neu-2 **(33b),** TN1-Neu-Mix (mixture of **33a** and **33b),** TN-1-NeuAc-1 **(29a),** TN-1-NeuAc-2 **(29b),** Sci-Neu-1 **(36a),** Sci-Neu-2 **(36b),** Sci-Neu(Mix) (mixture of **36a** and **36b**), Sci-NeuAc-1 **(32a),** and Sci-NeuAc-2 **(32b)]** were measured for their inhibitory activities against the sialidases of influenza viruses A/PR/8/34 (H1N1 subtype), A/Guizhou/54/89 (H3N2 subtype), and B/Ibaraki/2/85, at a final concentrations of 0.2 µg/mL, 1 µg/mL, 10 µg/mL, or 100 µg/mL, using 4-MU-NeuAc as a substrate and influenza HA vaccines as enzymes under each of the optimal pH conditions.

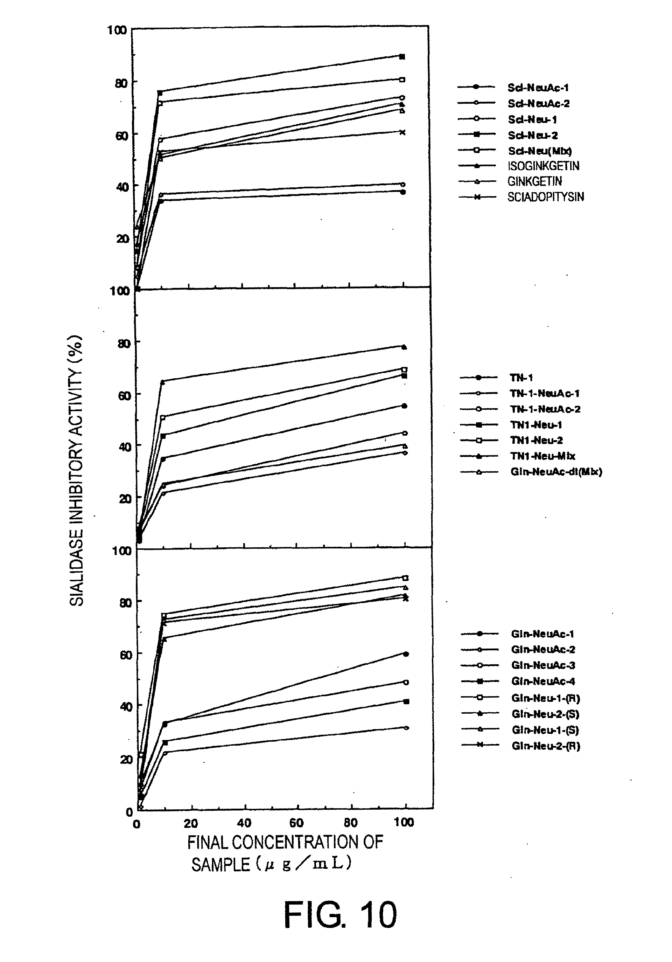

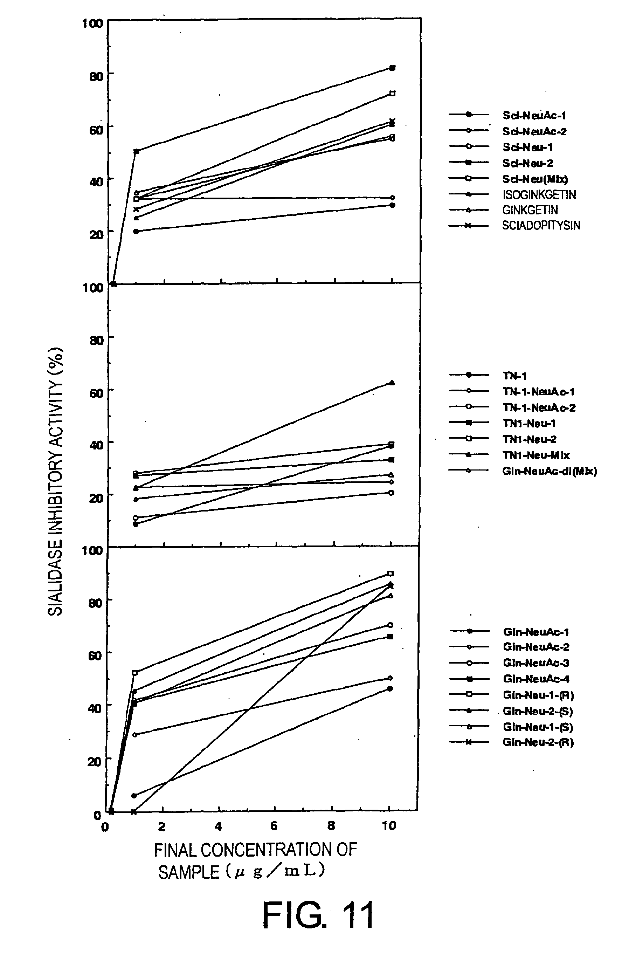

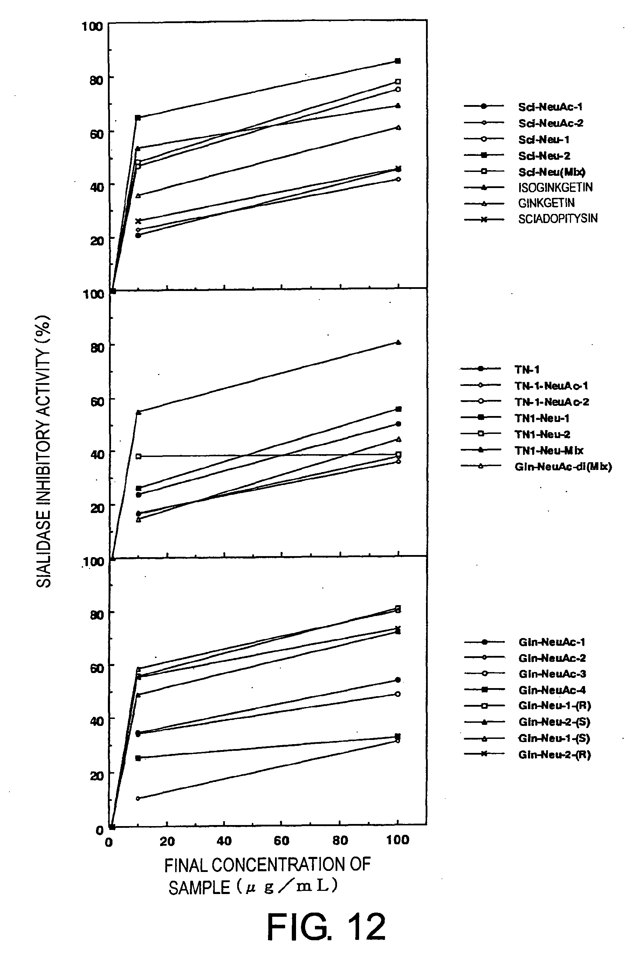

Results of the inhibitory activities against A/PR/8/34, A/Guizhou/54/89, and B/Ibaraki/2/85 virus sialidases are shown in Figs. 10, 11, and 12, respectively.

Among the samples tested, Sci-Neu-2, Gin-Neu-1-(R), Gin-Neu-2-(S), Gin-Neu-1-(S), and Gin-Neu-2-(R) showed 80% or more inhibitory activity against the A/PR/8/34 virus sialidase at a final concentration of 100 µg/mL. In addition, Sci-Neu-2, Sci-Neu(Mix), Gin-Neu-1-(R), Gin-Neu-1-(S), and Gin-Neu-2-(R) showed 70% or more inhibitory activity even at a final concentration of 10 µg/mL. When compared at the final concentration of 10 µg/mL, Sci-Neu-2 and Gin-Neu-1-(R) showed the highest inhibitory activity among the samples tested, and the 50% inhibitory concentrations (IC₅₀) of both were 5 µg/mL.

Against the A/Guizhou/54/89 virus sialidase, Sci-Neu-2, Gin-Neu-1-(R), Gin-Neu-2-(S), Gin-Neu-1-(S), and Gin-Neu-2-(R) showed 80% or more inhibitory activity at a final concentration of 10 µg/mL . In addition, six samples of Sci-Neu-2, Gin-NeuAc-3, Gin-NeuAc-4, Gin-Neu-1-(R), Gin-Neu-2-(S), and Gin-Neu-1-(S) showed about 50% inhibitory activity at a final concentration of 1 µg/mL. When compared at the final concentration of 1 µg/mL, Sci-Neu-2 and Gin-Neu-1-(R) showed the highest inhibitory activity among the samples tested, and the IC₅₀ of both was 1 µg/mL.

Against the B/Ibaraki/2/85 virus sialidase, Sci-Neu-2, TN1-Neu-Mix, Gin-Neu-1-(R), and Gin-Neu-1-(S) showed 80% or more inhibitory activity at a final concentration of 100 µg/mL. Six samples showed 50% or more inhibitory activity at a final concentration of 10 µg/mL, in which Sci-Neu-2 showed a high inhibitory activity of 65% (IC₅₀=7 µg/mL).

### [Example 4] In vitro anti-influenza virus activities

The same samples as those used in Example 3 (the four biflavones isolated from plants and the 19 biflavonoid-sialic acid conjugates prepared by organic synthesis) were measured for their *in vitro* anti-influenza virus activities. Specifically, MDCK (Madin-Darby canine kidney) cells cultured in a 96-well culture plate were infected with influenza A/PR/8/34 (H1N1 subtype) virus at a MOI (multiplicity of infection) of 0.001 in the presence of trypsin (3 µg/mL), and the sample (at a final concentration of 12.5 µg/mL) was added at the same time. The cells were incubated at 37°C for three days in a 5% CO₂ atmosphere. Amounts of viruses in the culture supernatant were measured as an indicator of anti-influenza virus activity by determining virus sialidase activity.

For cytotoxicity, in a similar way to when determining the anti-influenza virus activities, the test sample was added as described above to non-infected MDCK cells cultured in a 96-well culture plate. The cells were incubated at 37°C for three days in a 5% CO₂ atmosphere, and then cell viability was measured by the MTT method.

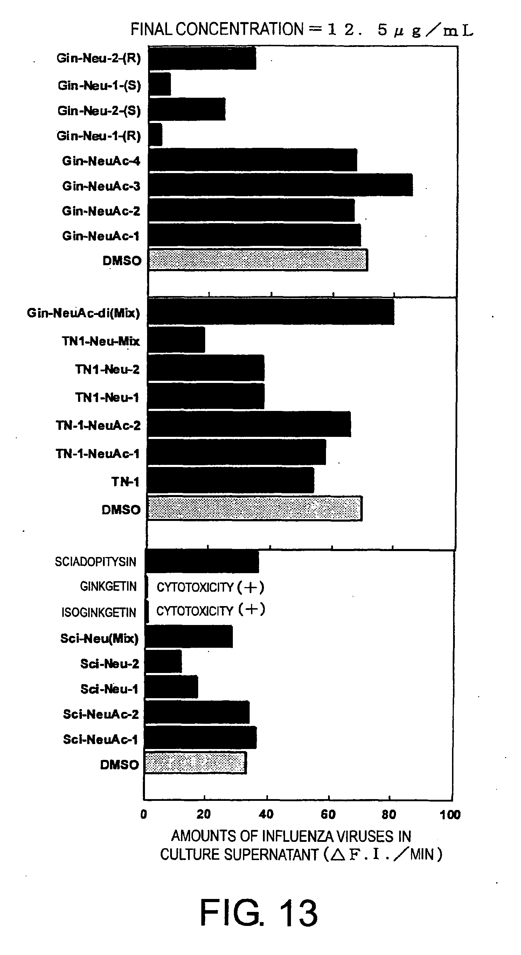

Fig. 13 shows the results. While ginkgetin (**20**) and isoginkgetin (**22**) showed cytotoxicity, the other tested samples did not. Using dimethylsulfoxide (DMSO) solvent as a reference, nine of the samples not showing cytotoxicity (Sci-Neu-1, Sci-Neu-2, TN1-Neu-1, TN1-Neu-2, TN1-Neu-Mix, Gin-Neu-1-(R), Gin-Neu-2-(S), Gin-Neu-1-(S), and Gin-Neu-2-(R)) produced a 45% or more decrease in the sialidase activity of the culture supernatant, indicating their activity in inhibiting influenza virus proliferation.

These results clearly indicate that biflavonoid-sialic acid conjugates not only exhibit higher *in vitro* anti-influenza virus activity, but also lower cytotoxicity than the original biflavones.

### [Example 5] In vivo anti-influenza virus activities

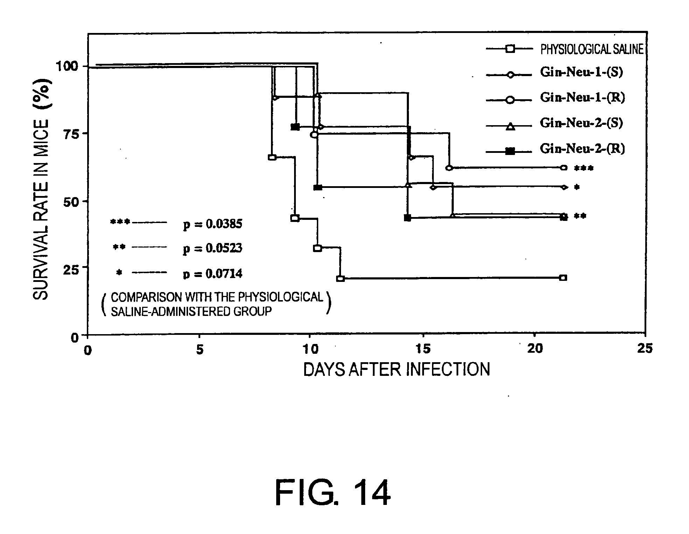

A stock solution of a mouse-adapted influenza A/PR/8/34 virus (50% lethal dose in mice (LD₅₀)=10^{4.83}) was 10^{4.5}-fold diluted (2x LD₅₀) with phosphate-buffered saline (PBS) containing a 0.1 % bovine serum albumin, to prepare a diluted virus suspension. BALB/c mice (female, seven weeks old) (CLEA Japan, Inc.) were anesthetized by an intraperitoneal injection of 0.25 mL amobarbital sodium solution (11 mg/mL in physiological saline), and infected with the virus through transnasal inoculation of 20 µL diluted virus solution. Each of the compounds [Gin-Neu-1-(R) **(34a),** Gin-Neu-1-(S) **(34b),** Gin-Neu-2-(R) **(35b),** and Gin-Neu-2-(S) **(35a)]** prepared in a similar way as described in Example 2 was dissolved in physiological saline to make up a 1 mg/mL solution, and administered once intranasally at a dose of 0.5 mg/kg to the mice anesthetized with amobarbital five minutes before inoculating the virus suspension. Physiological saline was intranasally administered instead of the compound solution to each of the mice in a control group. The survival rate of the mice was monitored for 21 days after infection of the influenza virus, and the *in vivo* anti-influenza virus activities of the compounds (Gin-Neu-1-(R), Gin-Neu-1-(S), Gin-Neu-2-(R), and Gin-Neu-2-(S)) were evaluated by comparing each survival rate with that of the control group. Fig. 14 shows the results, clearly indicating the followings.

The control group mice who received intranasal physiological saline began to die on day 8 after viral inoculation, and their survival rate from days 11 to 21 was 22%.

In contrast, the survival rate in mice who received intranasal compound Gin-Neu-1-(R) was 75% from days 10 to 16, and 62.5% even on day 21. Compared with the control group, a statistically significant increase in survival rate, and a prolonged survival time were observed (p=0.0385, Logrank test of the Kaplan-Meier method).

In addition, in mice who received intranasal compound Gin-Neu-2-(S), the survival rate was 89% from days 10 to 14, and 44% on day 21. Compared with the control group, statistical trends of increase in survival rate and prolonged survival time were observed (p=0.0523).

Moreover, in mice who received intranasal compound Gin-Neu-1-(S), the survival rate was 89% from days 8 to 10, 78% from days 10 to 14, and 56% on day 21. Compared with the control group, statistical trends of increase in survival rate and prolonged survival time were observed (p=0.0714).

In mice who received intranasal compound Gin-Neu-2-(R), the survival rate was 56% from days 10 to 14, and 44% on day 21. Compared with the control group, an increase in survival rate and a prolonged survival time were observed.

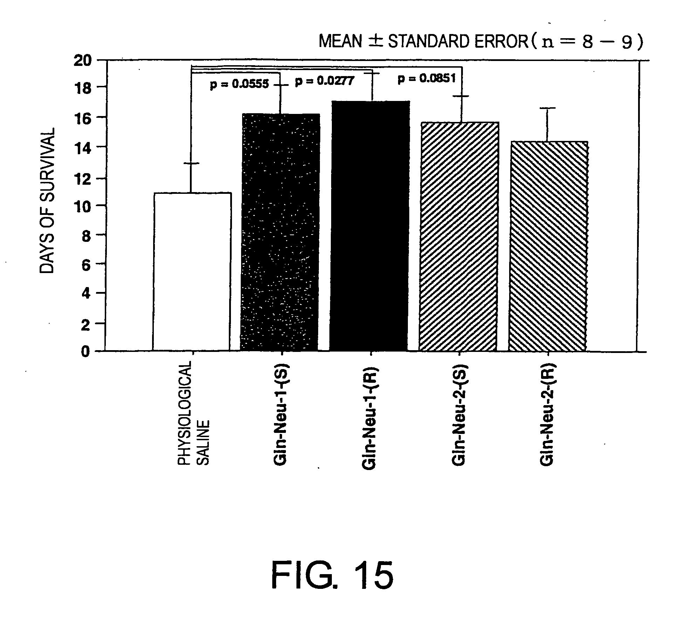

The mean survival time from these results was graphed in Fig. 15. As a result, the mean survival time was 10.9 days in the control group.

In contrast, in mice who received Gin-Neu-1-(R), the mean survival time was 17.3 days, which was 6.4 days longer than the control group, and was statistically significant (p=0.0277).

In addition, the mean survival time in mice who received Gin-Neu-1-(S) was 16.2 days, which was 5.3 days longer than the control group. A statistical trend to prolonged survival time was observed (p=0.0555).

Moreover, the mean survival time in mice who received Gin-Neu-2-(S) was 15.7 days, which was 4.8 days longer than the control group. A statistical trend to prolonged survival time was observed (p=0.0851). The mean survival time in mice who received Gin-Neu-2-(S) was 14.3 days, which was 3.4 days longer than the control group.

These results clearly indicate that the compounds (Gin-Neu-1-(R), Gin-Neu-1-(S), Gin-Neu-2-(R), and Gin-Neu-2-(S)) show anti-influenza virus activities, even in *in vivo* systems.

### [Example 6] Production of a biflavonoid-sialic acid conjugates that contains sulfur atoms (1) Synthesis of a SAc derivative of sialic acid

5.32 g of the sialic acid derivative (25) synthesized in Example 2 was dissolved in 60 ml of CH₂Cl₂ (dry) and 3.5 g of AcSK (potassium thioacetate) was added to the solution. The mixture was stirred in an argon gas atmosphere for 18 hours. After the reaction was finished, the reaction mixture was extracted with CHCl₃ and washed with saturated NaCl solution and saturated NaHCO₃ solution, in that order. The organic layer was dried over anhydrous Na₂SO₄, followed by vacuum concentration. This was purified by silica gel column chromatography (CHCl₃-MeOH), yielding the sialic acid derivative (IV-5)
(methyl-4,7,8,9-tetra-O-acetyl-2-*S*-acetyl-*N*-acetylneuraminate) (37) (3.62 g, 63%), which is represented by formula (IV) mentioned above, in which R¹⁰, R¹¹, R¹², and R¹³ are acetyl groups, R⁹ is a methyl group, L is a SAc group, and X is an acetamino group.

### (2) Synthesis of the biflavonoid derivatives (Br forms)

Each of the biflavonoid derivatives (V), in which at least one of R²¹ A and R²⁰B is a hydroxy group, is dissolved in DMF(dry) and triphenylphosphine (one equivalent), and NBS (N-bromosuccinimide) (1 to 2 equivalents) are added to the solution. The solution is then stirred for 18 hours in an argon gas atmosphere. After the reaction is finished, the reaction mixture is extracted with CHCl₃, and washed with saturated NaCl solution and saturated NaHCO₃ solution, in that order. After the organic layer is dried over anhydrous Na₂SO₄, it is concentrated in vacuo. The Br forms of biflavonoid derivatives (V), in which at least one of R²¹ A and R²⁰B is Br, are obtained by purification from the concentrate using silica gel column chromatography or HPLC.

### (3) Synthesis of the conjugate

The Br form of the biflavonoid derivative (V) and sialic acid derivative (IV-5) mentioned above (1 to 2 equivalents) are dissolved in dry DMF, and diethylamine (0.5 to 1.0 equivalent) is added to the solution. The solution is then stirred for 18 hours in an argon gas atmosphere. After the reaction is finished, the reaction mixture is extracted with CHCl₃, and the organic layer is washed with saturated NaCl solution and saturated NaHCO₃ solution, in that order. The organic layer is dried over anhydrous Na₂SO₄, and then concentrated in vacuo. Silica gel column chromatography or HPLC is used to purify the protected form of the desired conjugate from the concentrate.

The protected conjugate is dissolved in MeOH (dry) and NaOMe (2 to 3 equivalents) is added to the solution. After stirring the solution for four hours, H₂O (10 to 15 equivalents=twice the amount of NaOMe) is added, and the solution is stirred for 18 hours in an argon gas atmosphere. After the reaction is finished, the reaction mixture is neutralized with 2N HCl, desalted using Sephadex LH-20 (MeOH) or HP-20 (H₂O→MeOH), and purified by ODS-HPLC to yield the desired conjugate.

### Industrial Applicability

The biflavonoid-sialic acid conjugates of the present invention show anti-influenza virus activities in both *in vitro* and *in vivo* systems, and are also very safe because they are derived from naturally occurring compounds. Thus, they are not only useful as preventive or therapeutic agents against influenza, but can also be applied to food and drink products for preventing or treating influenza.

## Claims

1. A biflavonoid-sialic acid conjugate.

2. The biflavonoid-sialic acid conjugate of claim 1, wherein the biflavonoid-sialic acid conjugate is a compound of any of formula (I), (II), or (III), or a salt thereof: 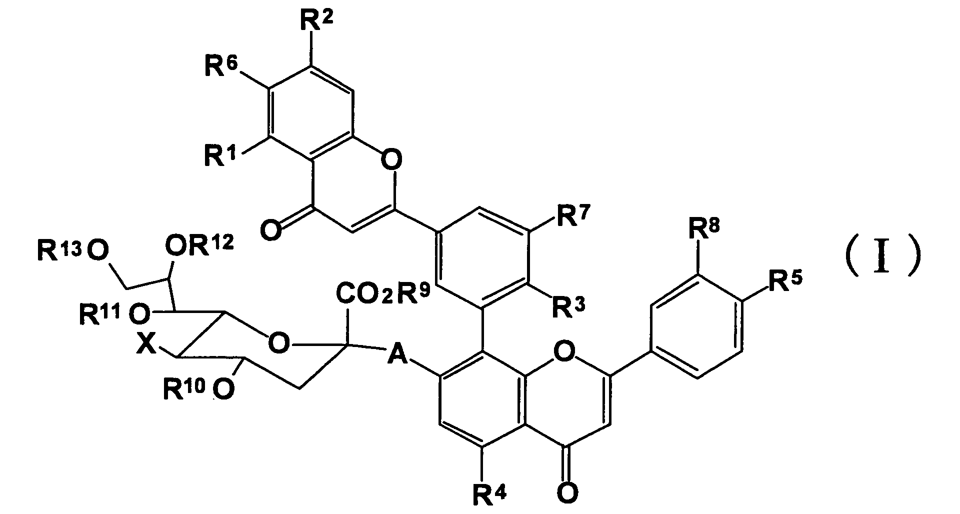 where in formula (1), R¹, R², R³, R⁴, R⁵, R⁶, R⁷, and R⁸ are each independently a hydrogen atom, methyl group, hydroxy group, acetyloxy group, methyloxy group, ethyloxy group, n-propyloxy group; n-butyloxy group, n-octyloxy group, benzyloxy group, allyloxy group, or glycosyloxy group, R⁹ is a hydrogen atom, sodium atom, potassium atom, ammonium group, or methyl group; R¹⁰, R¹¹, R¹², and R¹³ are each independently a hydrogen atom, sulfate group, or acetyl group; X is an acetamino group, glycolylamino group, hydroxy group, or acetyloxy group; and A is an oxygen atom or sulfur atom; 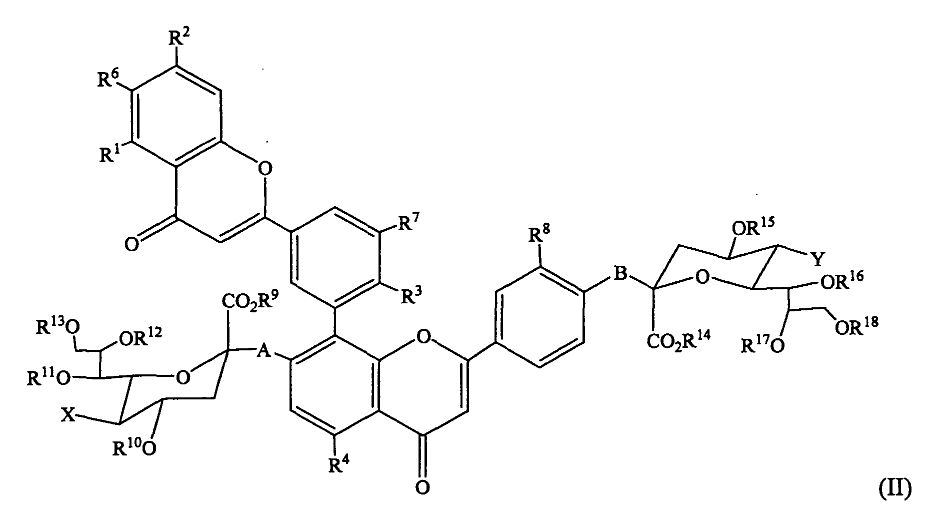 where in formula (II), R¹, R², R³, R⁴, R⁶, R⁷, and R⁸ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group; R⁹ and R¹⁴ are each independently a hydrogen atom, sodium atom, potassium atom, ammonium group, or methyl group; R¹⁰, R¹¹, R¹², R¹³, R¹⁵, R¹⁶, R¹⁷, and R¹⁸ are each independently a hydrogen atom, sulfate group, or acetyl group; X and Y are each independently an acetamino group, glycolylamino group, hydroxy group, or acetyloxy group; and A and B are each independently an oxygen atom or sulfur atom; and 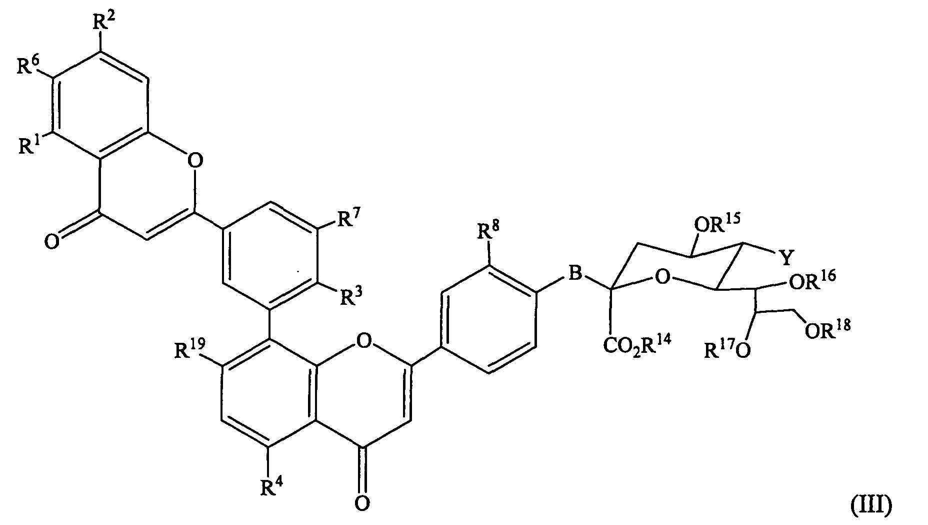 where in formula (III), R¹, R², R³, R⁴, R⁶, R⁷, R⁸, and R¹⁹ are each independently a hydrogen atom, methyl group, hydroxy group, acetyloxy group, methyloxy group, ethyloxy group, n-propyloxy group, n-butyloxy group, n-octyloxy group, benzyloxy group, allyloxy group, or glycosyloxy group; R¹⁴ is a hydrogen atom, sodium atom, potassium atom, ammonium group, or methyl group; R¹⁵, R¹⁶, R¹⁷, and R¹⁸ are each independently a hydrogen atom, sulfate group, or acetyl group; Y is an acetamino group, glycolylamino group, hydroxy group, or acetyloxy group; and B is an oxygen atom or sulfur atom.

3. The biflavonoid-sialic acid conjugate of claim 2, where in formulae (I) to (III), R⁹, R¹⁰, R¹¹, R¹², R¹³, R¹⁴, R¹⁵, R¹⁶, R¹⁷, and R¹⁸ are hydrogen atoms.

4. A pharmaceutical agent comprising the biflavonoid-sialic acid conjugate of any one of claims 1 to 3 as an active ingredient.

5. A preventive agent or a therapeutic agent for influenza, comprising the biflavonoid-sialic acid conjugate of any one of claims 1 to 3 as an active ingredient.

6. A food or drink product comprising the biflavonoid-sialic acid conjugate of any one of claims 1 to 3 as an active ingredient.

7. A food or drink product for preventing or treating influenza comprising the biflavonoid-sialic acid conjugate of any one of claims 1 to 3 as an active ingredient.
